(19) **Europäisches Patentamt / European Patent Office / Office européen des brevets**

(11) **EP 2 795 498 B1**

(12) # EUROPEAN PATENT SPECIFICATION

(45) Date of publication and mention
of the grant of the patent:
**07.11.2018 Bulletin 2018/45**

(21) Application number: **12815690.8**

(22) Date of filing: **20.12.2012**

(51) Int Cl.:
*G06F 19/16* [(2011.01)]    *G06F 19/00* [(2018.01)]

(86) International application number:
**PCT/EP2012/076500**

(87) International publication number:
**WO 2013/092922 (27.06.2013 Gazette 2013/26)**

(54) **METHOD OF BINDING SITE AND BINDING ENERGY DETERMINATION BY MIXED EXPLICIT SOLVENT SIMULATIONS**

VERFAHREN ZUR BINDUNGSSTELLEN- UND BINDUNGSENERGIEBESTIMMUNG DURCH GEMISCHTE EXPLIZITE LÖSUNGSMITTELSIMULATIONEN

PROCÉDÉ DE DÉTERMINATION D'UN SITE DE LIAISON ET D'UNE ÉNERGIE DE LIAISON PAR SIMULATIONS DE SOLVANTS EXPLICITES MÉLANGÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.12.2011 EP 11380102**

(43) Date of publication of application:
**29.10.2014 Bulletin 2014/44**

(73) Proprietors:
• **Universitat de Barcelona**
  08028 Barcelona (ES)
• **Institució Catalana de Recerca i Estudis Avançats**
  08010 Barcelona (ES)

(72) Inventors:
• **BARRIL ALONSO, Xavier**
  43812 Montferri (ES)
• **ALVAREZ GARCIA, Daniel**
  08224 Terrassa (ES)
• **SCHMIDTKE, Peter**
  92120 Montrouge (FR)

(74) Representative: **ZBM Patents - Zea, Barlocci & Markvardsen**
  **Plaza Catalunya, 1**
  **08002 Barcelona (ES)**

(56) References cited:
WO-A2-2010/129386

• **BAYNES B. M. ET AL: "Proteins in Mixed Solvents: A Molecular-Level Perspective", THE JOURNAL OF PHYSICAL CHEMISTRY B, vol. 107, no. 50, 1 December 2003 (2003-12-01), pages 14058-14067, XP055026097, ISSN: 1520-6106, DOI: 10.1021/jp0363996**
• **JIAO D. ET AL: "Trypsin-ligand binding free energies from explicit and implicit solvent simulations with polarizable potential", JOURNAL OF COMPUTATIONAL CHEMISTRY, vol. 30, no. 11, 1 August 2009 (2009-08-01), pages 1701-1711, XP055026198, ISSN: 0192-8651, DOI: 10.1002/jcc.21268**
• **DENG Y. ET AL: "Computations of Standard Binding Free Energies with Molecular Dynamics Simulations", THE JOURNAL OF PHYSICAL CHEMISTRY B, vol. 113, no. 8, 26 February 2009 (2009-02-26), pages 2234-2246, XP055026197, ISSN: 1520-6106, DOI: 10.1021/jp807701h**
• **INOUE H. ET AL: "Preferential and absolute interactions of solvent components with proteins in mixed solvent systems", BIOPOLYMERS, vol. 11, no. 4, 1 April 1972 (1972-04-01), pages 737-743, XP055026555, ISSN: 0006-3525, DOI: 10.1002/bip.1972.360110402**

## Description

FIELD OF THE INVENTION

The present invention relates to a method of computational chemistry used in rational drug design and receptor-ligand interaction modelling. In particular, to a method of *in silico* molecular design based on explicit solvent all-atom simulations that allow computing corrected interaction maps and free energies of binding which can be used in finding novel ligands for a macromolecule.

BACKGROUND ART

Drugs are molecules that exert a therapeutic effect by binding or reacting with certain macromolecules of a host patient or a pathogenic organism, activating or inhibiting certain processes that are important in the development of disease. Rational drug design is based on finding out which macromolecules are related to a particular disease, and devising a focused strategy on modulating the behaviour of these macromolecules. For a drug to be able to bind or chemically react with a macromolecule the most important factor is complementarity. Drugs must be structurally and chemically complementary to the macromolecule they are expected to modulate. The degree of complementarity is very important, as it has an impact on the activity they have on the macromolecule under study, and consequently on the therapeutic effect they achieve.

For a molecule to become a drug, the degree of complementarity must be extreme. This explains why the processes of finding a chemical structure with promising activity (hit finding) on a receptor of interest, and optimizing its activity and physico-chemical properties (lead optimization) are very complex. It is statistically proven that for a given receptor, only a handful out of many hundreds of thousands of small molecules have promising activity (Hann MM. et. al. "Molecular complexity and its impact on the probability of finding leads for drug discovery" J. Chem. Inf. Comput. Sci. 2001, vol. 41, pp. 856-864). This promising activity must be optimized in a time-consuming and costly process.

The knowledge of the exact 3D structure of the macromolecule opens up venues for rational drug design and *in silico* modelling that can be applied with the hope of streamlining the discovery process. Computers can be used to screen large amounts of small molecules in search for the ones with the highest complementarity to the macromolecule. This is routinely done nowadays by applying docking calculations.

Unfortunately, docking calculations consider the biological macromolecular target as a rigid body, and only the conformational space of the small molecule is sampled during the calculations. This leads to a crude approximation, and generates inaccuracies that translate to both false positives and false negatives in virtual screening.

Target macromolecules and the solvent environment where they are found are dynamic. The phenomenon by which a probe molecule and the macromolecule adapt to each other is called induced fit. Both the macromolecule and the drug, thanks to being flexible, populate only a certain restricted area of conformational space upon binding. The same macromolecule can populate different conformations if exposed to different probe molecules. This is why application of molecular dynamics (MD) can be of high interest as compared to rigid body approximations. MD simulations represent the macromolecular target as a flexible body that can adapt itself to changes in its surrounding environment. Additionally, the solvent behaves dynamically and can populate a large set of configurations on the surface of the macromolecule in such a way that statistically meaningful values can be derived from its distribution. This, by itself, represents a major improvement over a single picture representation.

Usually the simulations are carried out in aqueous solution to be as close as possible to physiological conditions. However, water is a polar solvent, and thus it is difficult from a standard MD simulation run in water to extract any meaningful information about the way hydrophobic probe molecules react to the presence of, and interact with the solute (receptor). The affinity of drugs for the target proteins is largely explained by the competition between water and the organic molecule for the site, and this phenomenon cannot be studied if only one of the components is present. All that can be done in a molecular dynamics simulation run in water is to extract preferential sites of interaction for a hydrogen bond donor and a hydrogen bond acceptor, the only two interaction features found in a water molecule. However, drugs and their precursors are molecules composed of both hydrophilic and hydrophobic functional groups. Furthermore, it is known that the hydrophobic effect is a very important driving force for the formation of the receptor-ligand complex (Orozco M., Luque FJ. "Theoretical methods for the description of the solvent effect in biomolecular systems" Chem. Rev. 2000, vol. 100, pp. 4187-4226). A great boost in activity can be achieved when a hydrophobic probe displaces water molecules solvating a hydrophobic site of the receptor macromolecule due mainly to entropy effects. Clues on the location of such sites are very difficult to be obtained by a simulation where the macromolecule is solely solvated in water. The binding sites of some receptors have such geometry and hydrophobicity that partially or even totally preclude the presence of water molecules. This has been seen in targets of high pharmacological importance such as cyclooxygenase-2 (Cox-2) and Human Serum Albumin (Young T., et. al. "Dewetting transitions in protein cavities" Proteins 2010, vol. 78, pp. 1856-1869). For these cases, the generation of binding free energy maps derived from a molecular dynamics trajectory of a receptor run exclusively in a water solution is especially challenging (Young T., et.al. "Motifs for molecular recognition exploiting hydrophobic enclosure in protein-ligand binding" PNAS 2006, vol. 104, pp. 808-813).

The mentioned limitations can be overcome by running MD simulations in a mixed solvent environment, where there is a polar solvent such as water, and a hydrophobic one that can engage the macromolecule via a hydrophobic or polar interaction, thus displacing water molecules. This can be achieved by placing a number of copies of the hydrophobic solvent among the water molecules prior to running the simulation, in a number that allows acquiring a fixed concentration of the organic solvent. In principle, this would allow all interesting parts of the surface of the macromolecule to be exposed to both polar and hydrophobic chemical groups, and either of the two could freely engage the protein in preferred locations once the system has reached a dynamic equilibrium. However, hydrophobic co-solvents tend to aggregate. This leads to inaccuracies and artefacts generated during the simulations, as the hydrophobic probe molecules orient in such a way as to minimize the contacts of their hydrophobic parts with water molecules, and thus are not evenly distributed over the simulated macromolecule.

One of the strategies recently implemented overcomes the aggregation problem by applying an artificial repulsive force among all hydrophobic solvent molecules during simulation. Thus, the SILCS methodology has been disclosed (WO 2010/129386 A2), where explicit solvent MD simulations of proteins are carried out in a solvent composed of water plus benzene and propane molecules. The system is then simulated by molecular dynamics, and the 3 solvents are free to interact in any possible way, the only condition being imposed is the artificial repulsion between benzene:benzene, benzene:propane and propane:propane in an effort to avoid their aggregation. The final result of this methodology is that differential solvation areas are found for each of the 4 atom types considered, i.e., hydrogen bond donor and acceptor (derived from water), aromatic carbon (derived from benzene) and aliphatic carbon (derived from propane).

From what is known in the art, there remains a need for alternative methods that can also derive preferential interaction sites and calculate binding free energies from a simulation, both for polar, ionic and hydrophobic chemical groups. This is an active area in rational drug design, and faster, more accurate and universally applicable methods are still being pursued.

SUMMARY OF THE INVENTION

The invention disclosed herein is a method to identify preferential binding sites on the surface of a macromolecule and to determine the free energy of binding to those sites that uses statistical information derived from simulations run in explicit mixed solvent. The same macromolecule is immersed in different solvent mixtures, which are composed of water and at least one other amphiphilic organic co-solvent. By amphiphilic, it is meant that the co-solvent molecules have a polar head combined with a hydrophobic tail. The use of amphiphilic organic molecules as co-solvents allows to apply the method disclosed herewith without resorting to apply any additional artificial forces to the mixed solvent simulations, as the amphiphiles do not lead to a separation of phases as there are when the co-solvents are totally hydrophobic (for instance benzene). Because the organic co-solvents used bear both polar and hydrophobic chemical groups, their binding to the surface of the macromolecule is a combination of the interaction of both groups (hydrophobic and hydrophilic). This must be taken into account, so that the binding of the polar head is decoupled from the binding of the hydrophobic tail when calculating the free energies of binding. This is done so by applying the correction that forms part of the invention disclosed herewith, by which when calculating the free energy of binding of one part of the organic co-solvent (head or tail) the binding of the other part is substracted. The strategy to apply the decoupling is to take as reference the interaction free energy of the whole co-solvent molecule, redistributing the atomic free energies of binding. For instance, if the co-solvent used together with water for solvating the macromolecule is isopropanol, this organic solvent can interact simultaneously with the macromolecule via its hydroxyl polar head and its hydrophobic isopropane tail. The two interactions must be decoupled in the calculation of the real free energies of binding of the different atom types that form isopropanol. If decoupling is not considered, the energy of binding of the hydroxyl head might be erroneously taken into account when calculating the binding energy of the isopropane tail and *vice versa.*

The advantages of applying the disclosed methodology are manifold.

1) No artificial forces are applied to any of the solvent molecules, which are free to reach an equilibrium both in the presence and absence of a receptor.
2) There is no separation of phases, as the organic solvents used always bear a polar head and form homogeneous mixtures in aqueous environments.
3) A variety of amphiphilic solvents can be used together with water. Many of them will have different functional groups. From their corrected distributions, the free energy of binding of multiple different atom types can be derived, ensuring that the scoring function to be built later on can have as many atom types as needed to properly cover the chemical diversity of a typical molecular database used for *in silico* hit finding purposes.
4) Related to the last point, the corrected atomic free energies of binding are comparable, allowing a fair comparison between features or atom types. This feature is not possible when the correction is not applied as, depending on the composition of the solvent molecule, some of the atom types may be overestimated.

5) Some of the co-solvents can have a net charge different to zero. Thus, in cases where the macromolecular receptor is suspected to be interacting with ionized groups, the simulations can be run with charged amphiphilic co-solvents such as methylammonium and acetate. In this way, the corrected free energies of binding of charged groups can also be extracted from the simulations.

6) The method is parameter-free, that is, there is no need to run any parameterization simulations, since no parameters are used for applying the invention.

By running these simulations, and subsequently applying the decoupling correction, more accurate free energy profiles can be obtained. Thus, the methodology allows for the first time to derive corrected free energies of binding of the most representative atom types from simulations where a mixture of water plus other organic solvents freely interact with the receptor. The inventors have surprisingly found that these corrected free energies of binding give superior results in the docking calculations routinely applied in drug discovery as compared to the uncorrected free energies of binding and the scoring functions typically used. Further, the inventors have also found that using the corrected free energies generated by the invention in docking and scoring calculations give absolute free energies of binding to a macromolecular target that deviate much less from the experimental values than those obtained by the uncorrected values or other techniques widely in use.

By running multiple simulations of the same receptor in which water is combined with different amphiphilic solvents in each case, and applying the decoupling correction forming part of the invention, one can derive free energies of binding for many different atom types. These free energies of binding can later on be incorporated to a scoring function to be used in docking calculations, and thus can be applied in different phases of the drug discovery process such as *in silico* hit finding of molecular databases, binding mode determination of chemical cores, and lead optimization.

Complementarily, the contours derived from the corrected probability distributions of the different atom types can also be used qualitatively to guide the design process of new ligands when examined in a computer graphics system. Thus in an exercise of computer-aided design, an overlap of a compound with known binding mode to the reference image of the receptor where areas of preferential interaction for the different atom types are visualized can give clues as to what chemical groups might be attached to the compound in further rounds of chemical synthesis in order to generate more active derivatives. Additionally, the contours derived from the invention can also be used to build more accurate three dimensional pharmacophores.

Thus, one aspect of the invention is a method of computational chemistry for calculating binding free energy/ies of a molecule binding to a macromolecular receptor using data derived from classical simulations, which are carried out in a mixed explicit solvent, comprising: a) inputting the 3D structure of a macromolecular receptor in a computer system; b) building at least one co-solvated simulation system comprising the macromolecular receptor and a mixture of explicit solvents comprising water and at least one organic amphiphilic co-solvent; c) assigning atom types to the atoms in the solvent molecules of the at least one co-solvated simulation system of step b); d) calculating at least one trajectory of the at least one co-solvated simulation system; e) partitioning the at least one co-solvated simulation system into volume elements; f) counting the presence of each solvent atom type in each volume element along the at least one trajectory of the at least one co-solvated simulation system to give the total occupancy over the simulated time in the volume element; g) using the results in the last step for obtaining free energies of binding to the macromolecule of the different atom types found in the solvent molecules; h) decoupling the contribution of each atom type in the co-solvent molecule from the rest of atom types of the co-solvent molecule to obtain corrected free energies of binding of the atom type in each volume element by carrying out a step selected from the group consisting of: h1) in step f) redistributing the count units amongst the atom types of the co-solvent molecule so that the count assigned to the atom type is directly proportional to its binding free energy contribution;

and, h2) in step g) subtracting the binding free energy due to all the other atom types present in the co-solvent molecule from the uncorrected binding free energy of the atom type. i) gathering all the corrected free energies of binding obtained in step h) for all the atom types to build a corrected scoring function; and j) based on docking, calculating the binding free energy of the molecule binding to the macromolecular receptor using the corrected scoring function obtained in step i). Another aspect of the invention is a method of computational chemistry for calculating binding free energy/ies of a molecule binding to a macromolecular receptor using data derived from classical simulations, which are carried out in a mixed explicit solvent, comprising: a) inputting the 3D structure of a macromolecular receptor in a computer system; b) building at least one co-solvated simulation system comprising the macromolecular receptor and a mixture of explicit solvents comprising water and at least one organic amphiphilic co-solvent; c) assigning atom types to the atoms in the solvent molecules of the at least one co-solvated simulation system of step b); d) calculating at least one trajectory of the at least one co-solvated simulation system; e) partitioning the at least one co-solvated simulation system into volume elements; f) counting the presence of each solvent atom type in each volume element along the at least one trajectory of the at least one co-solvated simulation system to give the total occupancy over the simulated time in the volume element; g) using the results in the last step for obtaining free energies of binding to the macromolecule of the different atom types found in the solvent molecules; h) decoupling the contribution of each atom type in the co-solvent molecule from the rest of atom types of the co-solvent molecule to obtain corrected free energies of binding of the atom type in each volume element by carrying out a step selected from the group consisting of: h1) in step f) redistributing the count units amongst the atom types of the co-solvent molecule so that the count assigned to the atom type is directly proportional to its binding free energy contribution; and h2) in step g) subtracting the binding free energy due to all the other atom types present in the co-solvent molecule from the uncorrected binding free energy of the atom type. i) gathering all the corrected free energies of binding obtained in step h) for all the atom types to build a corrected scoring function; and j) based on docking, calculating the binding free energy of the molecule binding to the macromolecular receptor using a scoring function, and rescoring the docked binding molecule using the corrected scoring function obtained in step i).

Another aspect of the invention relates to a computer program product comprising program instructions for causing a computer to perform the method of computational chemistry for calculating binding free energy/ies of a molecule binding to a macromolecular receptor as defined above. The computer program may be embodied on storing means (for example, on a record medium, on a computer memory or on a read-only memory) or carried on a carrier signal to be, for example, downloaded from a computer or sent by an email (for example, on an electrical or optical carrier signal).

Another aspect of the invention relates to a system of computational chemistry for calculating binding free energy/ies of a molecule binding to a macromolecular receptor using data derived from classical simulations, which are carried out in a mixed explicit solvent, comprising: a) computer means for inputting the 3D structure of a macromolecular receptor in a computer system; b) computer means for building at least one co-solvated simulation system comprising the macromolecular receptor and a mixture of explicit solvents comprising water and at least one organic amphiphilic co-solvent; c) computer means for assigning atom types to the atoms in the solvent molecules of the at least one co-solvated simulation system of step b); d) computer means for calculating at least one trajectory of the at least one co-solvated simulation system; e) computer means for partitioning the at least one co-solvated simulation system into volume elements; f) computer means for counting the presence of each solvent atom type in each volume element along the at least one trajectory of the at least one co-solvated simulation system to give the total occupancy over the simulated time in the volume element; g) computer means for using the results in the last step for obtaining free energies of binding to the macromolecule of the different atom types found in the solvent molecules; h) computer means for decoupling the contribution of each atom type in the co-solvent molecule from the rest of atom types of the co-solvent molecule to obtain corrected free energies of binding of the atom type in each volume element by carrying out a step selected from the group consisting of: h1) in step f) redistributing the count units amongst the atom types of the co-solvent molecule so that the count assigned to the atom type is directly proportional to its binding free energy contribution; and, h2) in step g) subtracting the binding free energy due to all the other atom types present in the co-solvent molecule from the uncorrected binding free energy of the atom type; i) computer means for gathering all the corrected free energies of binding obtained in step h) for all the atom types to build a corrected scoring function; and j) computer means for based on docking, calculating the binding free energy of the molecule binding to the macromolecular receptor using the corrected scoring function obtained in step i).

It is important to highlight that the described system can be a part (for example, hardware in the form of a PCI card) of a computer system (for example a personal computer). On the other hand, the system can be an external hardware connected to the computer system by appropriate means.

BRIEF DESCRIPTION OF THE DRAWINGS

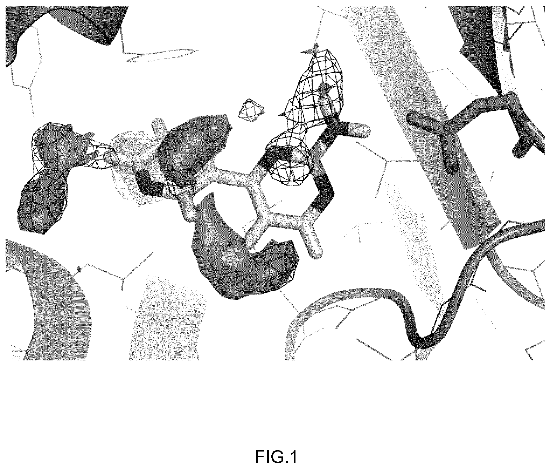

FIG. 1 Binding site of Hsp90 with a representative ligand (PDB code 2XDK) displayed as reference. Isocontours delimiting the most favourable interaction volumes for the methyl of isopropanol (hydrophobic probe) are represented as a mesh for the uncorrected free energy grid obtained with formula (1) ($\Delta G_{bind}$ isovalue of -1.5 kcal/mol) and as a transparent surface for the corrected free energy grid obtained with formula (6) with $\alpha$ values obtained from equation (5) ($\Delta G_{bind}$ isovalue of -0.7 kcal/mol). It can be appreciated: i) that the isovalue surfaces encompass a similar value for 3 out of 5 hydrophobic surfaces; ii) that the corrected values are in better agreement with the maximal atomic contribution of real ligands (Kuntz ID., et.al. "The maximal affinity of ligands" PNAS 1999, vol. 96, pp. 9997-10002); iii) that a volume erroneously identified as hydrophobic in the uncorrected grid actually binds polar atoms (NH and N belonging to the amino-pyrimidine of the inhibitor are engulfed by the contour) and, proportionally, it is much more reduced after correction (it nearly disappears); and iv) that the same correction leads to an increase of hydrophobic volume in a nearby site.

DETAILED DESCRIPTION OF THE INVENTION

Definitions:

The terms used in the present application relate to computational chemistry, and are well-known to the person skilled in the art. However, some definitions have been included in order to avoid any misinterpretation.

The term "atom type" as used herein denotes an atom, optionally with attached hydrogen atoms, with characteristic chemical attributes. Said chemical characteristics are defined by its hybridization and may also be defined by its immediate bonded neighbours and the force field parameters. For example, the ethanol molecule may be considered to contain 3 atom types with distinct characteristics: OH (hydrogen bond donor and acceptor), CH3 (hydrophobic aliphatic), and CH2 (linker).

The term "counterion" as used herein denotes the ions that are placed in a system represented classically to cancel the net charge of a macromolecule, so that the whole system has a net charge of zero, and also to achieve a certain desired ionic strength. The most usual ions in molecular dynamics simulations are $Cl^-$ for anions and $Na^+$ for cations.

The term "classical" as used herein denotes a system where the atoms are represented as points in space. A classical representation is the alternative to a quantum mechanical representation where nuclear and electron positions are taken into account. Classical molecular representations are modelled with the help of force fields.

The term "equilibration" as used herein denotes a multi-step process by which the simulated system is taken from its initial state after building to a point where the system is ready for unrestrained molecular dynamics simulation. The process typically implies starting with low temperatures (for instance 100K) and tight restraints on a certain part of the system, usually the solute. The temperature is then gradually increased to reach room temperature, and the restraints are gradually removed so that the system is more and more free to move away from its initial position.

The term "explicit solvent" as used herein denotes a simulation where solvent and co-solvent molecules solvating the macromolecular receptor are represented explicitly, as opposed to implicit solvation where the molecules of solvent are not simulated, and solvent is only represented as a continuous medium.

The term "force field" as used herein denotes a set of equations that define the potential energy of the system as a function of atomic coordinates, and which are based on the use of parameters defining equilibrium bond lengths, angles, dihedral angles, atom charges, van der Waals radii, etc, derived from experimental data and quantum mechanical calculations that allow representing a system in a classical way, as opposed to quantum mechanically, where the representation of the system requires solving the wavefunction.

The term "minimization" as used herein denotes a calculation in which the atomic positions are optimized in order to find a local or a global minimum in the potential energy surface of a molecule.

The term "parameter" as used herein denotes a set of constants used in a force field to describe the behaviour of atoms, derived from quantum mechanical calculations or experimental data.

The term "pose" as used herein denotes the binding mode that a binding molecule adopts when bound to its macromolecular target. Poses of a binding molecule when bound to the macromolecular target can be generated by the use of docking programs.

The term "restraint" as used herein denotes a force exerted on an atom, a bond, an angle between 3 linked atoms, etc, that prevents it from deviating from a certain value. Restraints are used for example in the equilibration process of a molecular system, to ensure that the deviation with respect to the initial reference positions is not substantial. The stronger the force exerted, the less the atom location, bond, angle, etc. deviates from its original value.

The term "scoring function" as used herein denotes a set of fast approximate mathematical methods used to predict the strength of the non-covalent interaction between two molecules after they have been docked (typically, these two molecules are a macromolecular receptor linked to the development of a disease, and a small organic molecule such as a drug that is used to alter its activity).

The term "snapshot" as used herein denotes the picture of the molecular system at a certain point in time. It has the position and may also have the velocities of all atoms in a classical system.

The term "total occupancy" as used herein denotes the number of times a certain atom type falls within a specific volume element out of the number of times the program checks if there are any atoms within said volume element.

The term "trajectory" as used herein denotes the history of the positions and motions of all atoms in the system under study, generated by a sampling alogorithm.

Simulations carried out in explicit solvent give a richer picture of how solvent molecules react to the presence of the receptor, allow deriving statistical values on their distribution, and also represent friction between solute and solvent, something that cannot be achieved by implicit solvent models. The statistical values obtained on the distribution of solvent molecules over the surface of a receptor can later on be used to define free energies of binding to the different points of its surface, and thus used for building a scoring function with which to score the binding of organic molecules, something that can be used for finding new binders to the macromolecule, or to optimize the potency of known inhibitors.

Thus, in many instances the biological systems that are taken as targets on which rational drug design is applied are simulated in explicit solvent. Most nucleic acids and proteins, with the exception of those found embedded in a membrane, are found in aqueous solution, and therefore most of the times these macromolecules are simulated in water.

The usual steps taken by a person skilled in the art to simulate a macromolecular system in an aqueous solution are the following:

1. An initial structure of the target macromolecule, either experimentally determined or derived from computational means, must be loaded on a computer machine. This sometimes involves considering solely the solute (protein, nucleic acid, etc) and sometimes the solute plus some first shell solvent molecules that are considered important in terms of structure.

2. The structure might be first corrected. In the case of proteins, some loops might be missing because of limitations in the experimental techniques, the orientation of some residues such as asparagines, glutamines and histidines might be wrongly assigned in the experiment, and therefore need correction. Some aminoacids might be only partially solved, and thus must be completed as well.

3. The structure might need neutralization by the addition of counterions. Also, in many instances more counterions are added than are needed to just neutralize the total charge of the system. This is done to simulate the receptor in an aqueous environment that has a certain ionic strength, as found in many cellular compartments.

4. The system is then solvated with water. The whole system can be simulated in Periodic Boundary Conditions (PBC) or in a spherical droplet of solvent.

5. Once the system is set up, it is typically minimized to relieve any steric clashes that might have been generated by the building process. For example, some solvent molecules might be too close to one another or to the solute, and this must be corrected before the next step.

6. The minimized system is then subjected to a series of restrained molecular dynamics simulations, where the temperature of the system is increased to usually reach 300 K. The first steps of the equilibration are carried out with very heavy restraints, that is, the positions of all atoms in the solute are heavily restrained, so that first the surrounding aqueous solution plus ions can freely move to lower energy locations. The next steps of the equilibration process are analogous, but the restraints become more and more permissive, so that the receptor can also adapt to its surroundings.

7. Once the system is considered equilibrated, unrestrained molecular dynamics simulations can be run in which the whole system is free to flexibly move and evolve during a time window. The pictures (snapshots) taken of the whole system during this period make the trajectory, which gives the positions and velocities of all atoms in the system (both solute and solvent). Typically, the control of the Root Mean Square Deviation (RMSd) of all atoms over time with respect to their initial positions allows to see the deviation of the system as a function of time, and also allows one skilled in the art to know whether the system has reached an equilibrium (the solute atoms are dynamically moving around a certain equilibrium position). This is the most interesting state, as it is the state that is expected to follow a Boltzmann distribution.

After running a simulation, all the snapshots can be placed over the same reference frame to cancel the drifting of the macromolecule during the simulation time. The whole system is then placed on a grid encompassing the whole simulation box. One skilled in the art can then count the number of times a solvent atom type falls within a certain volume element ($N_i$). Comparing it with the expected number of times that the atom type falls in the same volume of a homogeneous solvent box ($N_o$), the associated free energy of binding of said atom type in said volume element can be obtained by:

$$\Delta G = -k_B T \, Ln(N_i/N_o) \qquad (1)$$

where $k_B$ is the Boltzmann constant and T the temperature at which the system was simulated.

In this way, contours of preferential binding sites (higher than average probability) for different atom types can be obtained and visualized on a computer graphics system that give clues as to what chemical groups can fit what positions in the binding site of a receptor. Also free energies of binding of the atom type found in the solvent molecule that is analyzed can be derived. Thus, for instance if the macromolecule is solvated exclusively in water, and if one derives the number of times the water oxygen atom falls within volume element x, and having the average number of times a water oxygen falls within any grid element (derived from a water simulation), one can have the $\Delta G$ of binding of the atom type corresponding to water oxygen in volume element x by applying formula (1). By visualizing the probability of finding this atom type over the surface of the receptor, one can also have visual clues about where a hydrogen bond donor or acceptor should be placed, and this can be translated to further modifications to a molecule with incipient activity whose binding mode in the active site is known (for example adding a hydroxyl group overlapping one of the favourable contours derived from the water preferential binding sites).

Although the information derived from such simulations is very useful, the main limitation of this approach is the lack of other chemical features present in water, and thus the lack of $\Delta G$ of binding of other atom types that are very important in terms of molecular recognition. Importantly, it would be convenient if one could also derive the free energies of binding of other atom types derived from other types of solvent molecules. Ideally, these alternative solvent molecules should have hydrophobic groups that could engage the macromolecular receptor in hydrophobic interactions. In this way, the distribution of different types of solvent molecules would implicitly take into account the binding competition of polar and hydrophobic chemical groups found in the solvent for the different points on the macromolecular surface.

Because, as it has been explained above, simulations of totally hydrophobic co-solvents tend to lead to a separation of phases when mixed with a very polar solvent such as water, amphiphilic solvent molecules have been used instead, i.e. partly miscible solvents which bear a polar functional group coupled to a hydrophobic tail (such as isopropanol, acetamide, etc). These solvents can be used to compete with water to solvate the receptor surface and extract relevant data from their distribution, and no separation of phases takes place. For the application of the invention disclosed herewith, small organic co-solvents have been used, with few degrees of freedom (i.e. few rotatable bonds), as this ensures that these can easily diffuse on the surface of the receptor, and their distribution converges on the simulation time scale. However, these organic co-solvents can interact with the receptor via two different chemical features at once. The invention is based on the realization that the application of formula (1) to an organic co-solvent assumes that the distribution of the polar head is independent from that of the hydrophobic tail. However, a solvent organic molecule, such as isopropanol, can engage in a polar interaction with a polar group of the receptor at the same time as it engages in a hydrophobic interaction with an apolar spot of the protein via its hydrophobic tail. Thus, taking the two distributions as independent can lead to inaccuracies in the binding free energy calculation. The method disclosed herein has been found to effectively separate (decouple) the influence of the polar head from the influence of the hydrophobic tail. Inventors have found that the application of this method surprisingly allows to obtain more accurate absolute and relative free energies of binding.

Two means of implementing the method of the invention (decoupling) can be envisaged. A) Decoupling can be taken into account as a post-process of formula (1), or B) at the counting stage, i.e. before applying formula (1) :

A) decoupling might be based on the comparison between uncorrected $\Delta G$ of an atom type that belongs to a solvent molecule ($\Delta G_i^o$) and the $\Delta G$ of the whole molecule ($\Delta G_{WM}$), which might be represented by the centre of mass (CM) of that solvent molecule ($\Delta G_{CM}$). In an ideal case, if all the atoms of a solvent molecule contributed equally to binding, then each one would contribute $\Delta G_{CM}/N$ where N is the number of atoms in the solvent molecule. But because some atoms contribute more than others in many of the volume elements found on the surface of the receptor, that is, because some points on the surface of the macromolecule anchor the amphiphilic solvent predominantly thanks to the polar head and others thanks to the hydrophobic tail, the usual situation is that some atoms contribute more than average, while others contribute less in each volume element. In the former case, the $\Delta G_i$ of the atom will be lower than $\Delta G_{CM}/N$, while in the latter $\Delta G_i$ will be higher than $\Delta G_{CM}/N$. Thus, the present invention introduces a correction by which a fraction of the free energy of binding due to the whole molecule is subtracted from the free energy of binding due to a particular atom type in the co-solvent, and thus the final corrected free energy of binding for that atom is obtained. Many formulas can be devised to allow for the subtraction. As an example, one of the formulas tested to achieve this effect was:

$$\Delta G_i = \Delta G_i^o - \alpha \Delta G_{WM} \qquad (2)$$

where $\Delta G_i$ is the corrected free energy of binding of atom type i, and $\alpha$ can take any of the following expressions:

$$\alpha = (N-M)/N \qquad (3)$$

where N is the number of contributing atoms of the co-solvent molecule and M is the number of atoms corresponding to the atom type i.

$$\alpha = (SASA_{TOT} - SASA_i)/ SASA_{TOT} \qquad (4)$$

where $SASA_{TOT}$ is the total solvent accessible surface area of the co-solvent molecule and $SASA_i$ is the solvent accessible surface area of all atoms of type i.

$$\alpha = (\Delta G^{max}_{TOT} - \Delta G^{max}_i)/ \Delta G^{max}_{TOT} \qquad (5)$$

where $\Delta G^{max}_{TOT}$ is the maximum theoretical binding free energy of the co-solvent molecule and $\Delta G^{max}_i$ is the maximum theoretical binding free energy of atom type i. For polar atom types, $\Delta G^{max}_i$ can take a value of -1.8kcal/mol, as described in (Fersht AR. "The hydrogen bond in molecular recognition" Trends in Biochemical Science, vol.12, pp. 301-304). For apolar atom types, $\Delta G^{max}_i$ can be taken from (Karplus PA. "Hydrophobicity regained" Protein Science 1997, vol.6, pp. 1302-1307) as 0.025kcal per $A^2$ of the atom surface. The $\Delta G^{max}_{TOT}$ is the sum of $\Delta G^{max}_i$

If a certain point in the surface of the receptor is very favourably engaged only by the polar head of the co-solvent molecule, but there is nearly no interaction with the hydrophobic tail, then $\Delta G_{CM}$ will be close to zero, and the correction applied on $\Delta G_i^o$ to give $\Delta G_i$ will be very minor. However, if a certain point in the surface of the receptor is very favourably engaged by the polar head, but is next to a favourable site of interaction for the hydrophobic tail, which is also contributing to anchoring the solvent molecule, then the $\Delta G_{CM}$ will be high, as both head and tail will be in close contact with the receptor, and thus the correction will be significant. In this way, the influence of the hydrophobic tail in binding can be subtracted when calculating the binding of the polar head, and *vice versa.*

The method implies then the calculation of $\Delta G_{CM}$ to apply the correction. Because the CM can be in a number of volume elements when a certain solvent atom type is anchored within the same volume element, one needs to calculate the $\Delta G_{CM}$ of binding in each of those volume elements and the fraction of times the CM falls within each of those. Thus, the final corrected free energy value for each atom type of the co-solvent can be calculated by:

$$\Delta G_i \;=\; \sum_{j=1}^{m} f_j \Delta G_i^o - \alpha \Delta G_j^{CM} \qquad (6)$$

where m is the number of volume elements where CM is found, f is the fraction of times CM is found in each of those grid elements and N is the number of atoms the amphiphilic co-solvent is composed of.

In order to make the calculation of the CM less computationally demanding, a series of simplifications can be introduced to the method. Thus, for instance, in formula (6) using $\alpha$ values obtained from equation (3), if ethanol is used as co-solvent together with water, the CH2 linker can be taken as a surrogate for the centre of mass, and is supposed not to contribute to binding and therefore, for ethanol, N=2. For acetamide, acetone or acetate, the central carbon atom can be used as a CM and N can be taken to be 3.

B) decoupling can be performed at the counting step, to directly obtain corrected $\Delta G_{bind}$ values by applying formula (1). This implies that the count units are redistributed amongst the atoms of the co-solvent molecule in such a manner that those atoms contributing most to binding obtain a bigger share of the count units. In the ideal case of a molecule binding in a manner in which all atoms contribute equally to binding, the count units would be uniformly distributed. But because some atoms contribute more than others in many of the volume elements found on the surface of the receptor, the count units are usually unevenly distributed. For instance, in the case of a solvent molecule binding to the macromolecule mainly through the hydrophobic tail, the atoms in the polar head may get a count fraction close to zero, whereas atoms in the hydrophobic tail may get count values close to the total count units. In this manner, use of corrected counts in combination with formula (1) yields results similar to those obtained with formula (6). Many formulas can be devised for decoupling the atomic contributions at the counting step. As an example, one of the formulas tested to achieve this effect was:

$$C^i = N \left( E_{INTER}^i / E_{INTER}^{TOTAL} \right) \qquad (7)$$

where $E_{INTER}^i$ represents the interaction energy of atom type i with the macromolecular target (e.g. as obtained from a classical molecular force-field); $E_{INTER}^{TOTAL}$ represents the sum of $E_{INTER}^i$ over all atom types in the co-solvent molecule and N is the count units (e.g. 3 in the case of ethanol). Other partition schemes based on contact surface area or solvent-solvent interaction energy, amongst others, can be envisaged by one skilled in the art.

Applying this transformation to each snapshot in the trajectory, corrected counts per volume element ($N_i$) can be obtained using this formula:

$$N_i \;=\; \sum_{j=1}^{m} C_j^i \qquad (8)$$

where m is the number of snapshots in the trajectory. As in the uncorrected case, binding free energies can be obtained comparing ($N_i$) with the expectancy value ($N_o$) using equation (1). With the remarkable difference that, in this case, the obtained binding free energies can be considered corrected (decoupled).

The actual implementation of the present invention might be differently carried out depending on the macromolecule, as some of the simplifications might work better for some systems than for others. However, the actual simplification of some of the elements found would not deviate the method from the main idea of the invention. By carrying out a series of simulations with several amphiphilic solvents plus water, the corrected free energies of binding can be derived for different atom types. If these atom types are later on assigned to a wide series of molecules, then the free energies of binding can be calculated for all of them. In this way, a scoring function can be constructed and used for screening large virtual libraries in search of novel hits to the target under study. The molecules in the database can first be docked to the macromolecule. If all atoms in a n-th molecule can be assigned to the particular atom types whose corrected free energies of binding are derived from the mixed solvent simulations, then the global free energy of binding for n-th molecule can be derived, as a sum of the free energies of binding of its atoms.

Thus, as mentioned above, one aspect of the present invention is a method of computational chemistry for calculating binding free energy/ies of a molecule binding to a macromolecular receptor using data derived from classical simulations, where the simulations are carried out in a mixed explicit solvent, comprising the following steps:

a) inputting the 3D structure of a macromolecular receptor in a computer system. Typically, the systems to be simulated will be macro+ molecules such as proteins and nucleic acids that are interesting from a pharmacological point of view, and whose structures have been solved by experimental methods, such as X ray crystallography and Nuclear Magnetic Resonance (NMR). In the absence of such structure, the person skilled in the art can also resort to homology modelling if the 3D structure of a close homologue to the protein of interest is known;

b) building at least one co-solvated simulation system comprising the macromolecular receptor and a mixture of explicit solvents comprising water and at least one organic amphiphilic co-solvent. Once the structure is loaded in the computer, building the explicit solvent simulation system ensues by immersing the receptor in a solvent box, truncated octahedron or other geometric shapes, or alternatively in a solvent drop. Because the simulated system has a finite volume, one can place as many amphiphilic solvent molecules as it needs to reach a certain desired macroscopic concentration. At least one simulation of the macromolecule with water and at least one organic amphiphilic co-solvent is needed to apply the method of the invention, but typically several (2-8) simulations run in different solvent mixtures will be used, as can be seen in the examples provided.

c) assigning atom types to the atoms in the solvent molecules of the at least one co-solvated simulation system of step b). This is of especial importance, as the free energies of binding derived from the simulations will be assigned to each atom type (found in the different co-solvents) for each volume element, and will later on by transferred to all molecules to be modelled, so that their free energies of binding to the receptor can also be estimated. Although the most rigorous implementation of the method might be based on the assignment of atom types to the different co-solvent molecules, other partitioning schemes can also be used to divide the structure of the co-solvent molecule in different parts. For example, the method can also be implemented by dividing the co-solvent molecule into different probe structures, wherein the probe represents the assembly of two or more atom types, or the assembly of two or more atoms that have the same atom type.

d) calculating at least one trajectory of the at least one co-solvated simulation system . A series of different simulations can be run for the whole system. This is the production part of the simulation, where the solvent reacts to the presence of the solute, reaches an equilibrium and fluctuates dynamically around it. This window of the simulation is the one used to extract the data to be later on processed to obtain the free energies of binding. Calculation of the trajectories might be sometimes preceded by an equilibration phase, where the simulation is calculated with the help of restraints to alleviate some deficiencies associated to the building phase.

e) partitioning the at least one co-solvated simulation system into volume elements. All superimposed images of the system must be discretized so that statistics on the distribution of the different atom types, making up the different solvents, on the surface of the macromolecule can be derived. One of the strategies to carry out the latter would be to place all the snapshots taken from the simulations on a cubic grid with a fixed spacing, for example, 1 Angstrom. Other strategies not linked to the use of a grid, such as volumes related to atoms on the surface of the macromolecule, can also be implemented. Grid-based methods require to superimpose all the snapshots taken from the simulation, while other strategies might not require any superimposition.

f) counting the presence of each solvent atom type in each volume element along the at least one trajectory of the at least one co-solvated simulation system to give the total occupancy over the simulated time in the volume element. This step allows to obtain $N_i$ as discussed for formula 1 (found above).

g) Using the results in the last step for obtaining free energies of binding to the macromolecule of the different atom types found in the solvent molecules. This step can be typically done by applying formula (1) found above, but other means of extracting thermodynamic parameters may also be used (Ben-Naim, A., "Molecular theory of solutions", Oxford University Press, September 2006).

h) decoupling the contribution of each atom type in the co-solvent molecule from the rest of atom types of the co-solvent molecule to obtain corrected free energies of binding of the atom type in each volume element. This, as has been explained above, can be achieved by using one of these two strategies:

h1) in step f) redistributing the count units amongst the atom types of the co-solvent molecule so that the count assigned to the atom type is directly proportional to its binding free energy contribution. In this way, the atom type that contributes the most to binding to the macromolecule in the volume element is assigned a bigger count than the other atom types that do not contribute as much. The direct application of formula 1 found above to these counts allows to obtain corrected (decoupled) free energies of binding.

Alternatively, instead of decoupling at the counting stage, one can count all atom types equally, irrespective of their weight in terms of binding, but decouple the different contributions after formula 1 has been applied, by

h2) in step g) subtracting the binding free energy due to all the other atom types present in the co-solvent molecule from the uncorrected binding free energy of the atom type.

i) gathering all the corrected free energies of binding obtained in step h) for all the atom types to build a corrected scoring function. The determination of the corrected free energies of binding for all atom types in all volume elements surrounding the macromolecule allows one skilled in the art to derive a scoring function by standard techniques. In its simplest form, the score for a given binding mode of a ligand is the sum of corrected binding free energies of the corresponding atom types at the corresponding locations.

j) based on docking, calculating the binding free energy of the molecule binding to the macromolecular receptor using the corrected scoring function obtained in step i), or alternatively, based on docking, calculating the binding free energy of the molecule binding to the macromolecular receptor using a scoring function, and rescoring the docked binding molecule using the corrected scoring function obtained in step i). The corrected free energies of binding obtained in step h) can be used during the docking calculation as a fitness function for the optimization of the pose by which the binding molecule binds to the macromolecular receptor, or it can be used once the binding molecule has been docked to the macromolecular receptor, i.e. once the binding mode or pose of the binding molecule in the active site of the receptor has been determined (with any docking engine found in the art), in order to estimate the binding free energy related to that pose (rescoring).

In a preferred embodiment of the method of computational chemistry, in step j), the corrected scoring function is used for optimizing the binding mode of the binding molecule to the macromolecular receptor.

In another embodiment of the method of computational chemistry, in step j) the corrected scoring function is used, once the binding mode has already been determined by any *in silico* means, to determine the free energy of binding associated to that binding mode.

These last two embodiments highlight the fact that the corrected free energies of binding obtained by this method can be used for both the docking calculation and the scoring of the docked poses.

In a another embodiment of the method of computational chemistry of the invention, a local concentration correction is carried out prior to step g), comprising:

3-1) counting the number of contacts that each solvent molecule bearing the atom type present in the volume element makes with the rest of molecules belonging to the same type of solvent. Thus, if the amphiphilic solvent that is added to mix with water is for instance ethanol, and the aim is to derive the free energy of binding of its oxygen atom, one should count the number of times the ethanol oxygen falls in a certain volume element and then within that number, the number of times that the molecule is contacting other ethanol molecules.

3-2) running a series of simulations without any solute where there are different concentrations of the amphiphilic co-solvent plus water, to derive a relationship between global macroscopic concentration of the amphiphilic co-solvent and average number of contacts between the molecules of the amphiphilic co-solvent.

3-3) converting the number of contacts calculated in step 2-1) to the real concentration in each volume element by using the correspondence obtained in step 2-2). Thus, the number of contacts made by the amphiphilic solvent molecule to other molecules of its kind is the one that sets the real concentration in that volume element by using the reference concentrations previously calculated. That is, the concentration of the co-solvent is different in each volume element, not uniform for all of them, and it is converted by the number of contacts that the co-solvent establishes with itself along the simulation by using the relationship derived from step 2-2.

As an example, a range of simulations can be run for the isopropanol-water mixture at different concentrations of isopropanol: 2, 5, 10, 15, 30, 40, 70 and 85%. One can then count the average number of times the isopropanol molecule is contacting other isopropanol molecules in all those simulations. The number of times will obviously become bigger as the concentration of isopropanol is higher. This effectively allows having a direct relationship between number of contacts and macroscopic concentration. Then, one can count the number of times the oxygen of isopropanol falls within a certain volume element during a simulation of a receptor in an isopropanol-water mixture. By counting, not this number, but also the number of times the isopropanol molecule bearing the oxygen falling within said element is in contact with other isopropanol molecules, one can derive the concentration in said volume element by using the relationship found in the reference simulations run at different concentrations.

This local concentration correction when calculating the free energies of binding can have important consequences if the amphiphilic organic co-solvent used in the simulations together with water shows a strong tendency to aggregate. As it was explained above, the invention disclosed herein is based on analyzing simulations with water+amphiphilic solvents, not with totally hydrophobic ones such as benzene. However, among the organic solvents used for carrying out the simulations on which to apply the invention, some might have a greater tendency to aggregate when in the presence of a macromolecular receptor (for instance dimethylether aggregates more than isopropanol, due to its more hydrophobic nature). For all those organic solvents that show a strong tendency to aggregate, the local concentration correction is advisable.

In another preferred embodiment of the method of computational chemistry of the invention, the method can further comprise a step k) wherein visualization of the corrected free energy profiles of each atom type on the surface of the receptor is carried out in a computer graphics system. In this way, the corrected free energies coming from the application of the invention can, not only be used quantitatively in the building of a corrected scoring function, but also be qualitatively used to visualize areas of the active site where preferential spots for certain atom types are found, giving clues that can be used in the optimization of chemical series to improve their potency on the macromolecular receptor. Additionally, the contours can be used to build pharmacophores to summarize the main interactions of the active site of the macromolecule.

In another preferred embodiment of the method of computational chemistry of the invention, the calculation of the at least one trajectory of the at least one co-solvated simulation system is done with a molecular dynamics simulation. The trajectory giving the ensemble of structures of the co-solvated macromolecular receptor can be obtained by molecular dynamics simulations.

In another preferred embodiment of the method of computational chemistry of the invention, the calculation of the at least one trajectory of the at least one co-solvated simulation system is done with a MonteCarlo simulation. The trajectory giving the ensemble of structures of the co-solvated macromolecular receptor can also be obtained by MonteCarlo simulations.

In another preferred embodiment of the method of computational chemistry of the invention, the organic amphiphilic co-solvent is selected from the group consisting of a (C1-C8)-alcohol, a (C2-C8)-amide, a (C1-C8)-amine, a (C2-C8)-carboxylic acid, a (C2-C8)-ether, a (C2-C8)-thioether, a (C2-C8)-sulphone, a (C1-C8)-sulphonamide, a 5-7 membered saturated or unsaturated heterocycle containing S, O, or N, acetone, acetonitrile, urea, and a mixture of methylammonium and acetate, a (C1-C8)-alcohol substituted by one or more halogens, a (C2-C8)-amide substituted by one or more halogens, a (C1-C8)-amine substituted by one or more halogens, a (C2-C8)-carboxylic acid substituted by one or more halogens, a (C2-C8)-ether substituted by one or more halogens, a (C2-C8)-thioether substituted by one or more halogens, a (C2-C8)-sulphone substituted by one or more halogens, a (C1-C8)-sulphonamide substituted by one or more halogens, a 5-7 membered saturated or unsaturated heterocycle containing S, O, or N substituted by one or more halogens. By (C2-C8)-alcohol it is understood any co-solvent molecule bearing a polar hydroxyl head covalently attached to a hydrophobic tail containing 2 to 8 carbon atoms, either aromatic or aliphatic. The same applies to the other functional groups listed, even though some of them are found in the middle of a chain, not as an ending group in the molecule. Thus, by (C2-C8)-ether it is understood any ether bearing co-solvent molecule that has at both sides of the oxygen atom a total sum of a maximum of 8 carbons, and therefore both anisole and 1-methoxyheptane are included in the definition.

In a yet more preferred embodiment of the method of computational chemistry of the invention, the organic co-solvent is selected from the group consisting of ethanol, isopropanol, acetamide, pyridine, pyrimidine, piperazine, morpholine, pyrrole, oxazole, pyrazole, pyrrolidone, acetone, acetonitrile, trifluoroethanol, vinylamine, dimethylether, urea and a mixture of methylammonium and acetate.

In another preferred embodiment of the method of computational chemistry of the invention, step d) is performed with the application of Cartesian restraints on the receptor. Running the production part of the simulation with restraints applied on the macromolecular receptor might be advantageous as in this way the convergence of the simulation might be faster and more reproducible. Some of the macromolecular systems are very flexible, and can undergo great conformational changes within the simulation time. For reproducibility issues, it might be better to eliminate substantial conformational changes that only contribute to noise in the final result.

In another preferred embodiment of the method of computational chemistry of the invention, the macromolecular receptor is a protein or a glycoprotein.

In another preferred embodiment of the method of computational chemistry of the invention, the macromolecular receptor is a nucleic acid.

In another preferred embodiment of the method of computational chemistry of the invention, the macromolecular receptor is a carbohydrate.

In another preferred embodiment of the method of computational chemistry of the invention, there is a further step comprising adding missing atoms to the experimentally solved structure before step b). Some experimentally determined structures, such as proteins coming from X-ray crystallography, might lack some side chains in certain aminoacids, due to their high mobility (their electron density is simply not determined). It is always advisable to model missing side chains of all those aminoacids whose structures have only been partially determined experimentally, before solvating the macromolecule (i.e. before step b)).

In another preferred embodiment of the method of computational chemistry of the invention, there is a further step comprising assigning protonation states corresponding to physiological pH to the ionisable residues in the macromolecular receptor before step b). This can be very important, as sometimes the ionisable residues of a protein, such as aspartic and glutamic acid and lysine and arginine, have a net charge different to zero. This can have an important impact on the way some molecules bind the receptors, and also on the free energy of binding. Simulating the protein with these residues properly ionized might sometimes be key.

In another preferred embodiment of the method of computational chemistry of the invention, there is a further step comprising assigning the energetically preferred orientation of glutamine and asparagine residues and the energetically preferred tautomeric states to histidine residues before step b). Some experimental techniques used to elucidate the 3D structure of macromolecules such as X-ray crystallography are not able to properly place the oxygen and nitrogen atoms of an amide group such as the one found in asparagine and glutamine, because both O and N have approximately the same electron density. It might be important to correct some artefacts coming from this limitation before running the production part of the molecular dynamics simulation.

In another preferred embodiment of the method of computational chemistry of the invention, there is a further step comprising keeping all crystallographic water molecules and ions whose positions around the macromolecule have been determined experimentally before step b). Some macromolecules, for example some proteins, rely to maintain their structural integrity on the presence of certain solvent molecules, which are located at specific points on their surface or interior. These might be crucial in order to have a reliable and accurate simulated structure, and overlooking their presence might bias the simulation towards unrealistic structures. Thus, for some simulated systems, it is advisable to maintain the solvent molecules that have been determined experimentally together with the macromolecular structure.

In another preferred embodiment of the method of computational chemistry of the invention, the co-solvent molecules have a concentration from single molecule per simulated system to 80% v/v of the mixture.

In another preferred embodiment of the method of computational chemistry of the invention, the co-solvent molecules have a concentration from 5% to 30% v/v of the mixture.

Although the present invention has been described in detail for purpose of illustration, it is understood that such detail is solely for that purpose, and variations can be made therein by those skilled in the art without departing from the scope of the invention.

Thus, while the preferred embodiments of the methods and of the systems have been described in reference to the environment in which they were developed, they are merely illustrative of the principles of the invention. Other embodiments and configurations may be devised without departing from the scope of the appended claims.

Further, although the embodiments of the invention comprise processes performed in computer apparatus, the invention also extends to computer apparatus and to computer programs, particularly computer programs on or in a carrier, adapted for putting the invention into practice.

Accordingly, it also forms part of the invention a computer program product comprising program instructions for causing a computer to perform the method of computational chemistry for calculating binding free energy/ies of a molecule binding to a macromolecular receptor as defined above.

The program may be in the form of source code, object code, a code intermediate source and object code such as in partially compiled form, or in any other form suitable for use in the implementation of the processes according to the invention.

In a preferred embodiment, the computer program product is embodied on a storage medium.

In another preferred embodiment, the computer program product is carried on a carrier signal. The carrier may be any entity or device capable of carrying the program.

For example, the carrier may comprise a storage medium, such as a ROM, for example a CD ROM or a semiconductor ROM, or a magnetic recording medium, for example a floppy disc or hard disk. Further, the carrier may be a transmissible carrier such as an electrical or optical signal, which may be conveyed via electrical or optical cable or by radio or other means.

When the program is embodied in a signal that may be conveyed directly by a cable or other device or means, the carrier may be constituted by such cable or other device or means.

Alternatively, the carrier may be an integrated circuit in which the program is embedded, the integrated circuit being adapted for performing, or for use in the performance of, the relevant process.

As mentioned above, it also forms part of the invention a system of computational chemistry for calculating binding free energy/ies of a molecule binding to a macromolecular receptor using data derived from classical simulations, which are carried out in a mixed explicit solvent, comprising:

a) computer means for inputting the 3D structure of a macromolecular receptor in a computer system; b) computer means for building at least one co-solvated simulation system comprising the macromolecular receptor and a mixture of explicit solvents comprising water and at least one organic amphiphilic co-solvent; c) computer means for assigning atom types to the atoms in the solvent molecules of the at least one co-solvated simulation system of step b); d) computer means for calculating at least one trajectory of the at least one co-solvated simulation system; e) computer means for partitioning the at least one co-solvated simulation system into volume elements; f) computer means for counting the presence of each solvent atom type in each volume element along the at least one trajectory of the at least one co-solvated simulation system to give the total occupancy over the simulated time in the volume element; g) computer means for using the results in the last step for obtaining free energies of binding to the macromolecule of the different atom types found in the solvent molecules; h) computer means for decoupling the contribution of each atom type in the co-solvent molecule from the rest of atom types of the co-solvent molecule to obtain corrected free energies of binding of the atom type in each volume element by carrying out a step selected from the group consisting of: h1) in step f) redistributing the count units amongst the atom types of the co-solvent molecule so that the count assigned to the atom type is directly proportional to its binding free energy contribution; and h2) in step g) subtracting the binding free energy due to all the other atom types present in the co-solvent molecule from the uncorrected binding free energy of the atom type; i) computer means for gathering all the corrected free energies of binding obtained in step h) for all the atom types to build a corrected scoring function; and j) computer means for based on docking, calculating the binding free energy of the molecule binding to the macromolecular receptor using the corrected scoring function obtained in step i).

It is also considered a part of the invention a system of computational chemistry for calculating binding free energy/ies of a molecule binding to a macromolecular receptor using data derived from classical simulations, which are carried out in a mixed explicit solvent, comprising: a) computer means for inputting the 3D structure of a macromolecular receptor in a computer system; b) computer means for building at least one co-solvated simulation system comprising the macromolecular receptor and a mixture of explicit solvents comprising water and at least one organic amphiphilic co-solvent; c) computer means for assigning atom types to the atoms in the solvent molecules of the at least one co-solvated simulation system of step b); d) computer means for calculating at least one trajectory of the at least one co-solvated simulation system; e) computer means for partitioning the at least one co-solvated simulation system into volume elements; f) computer means for counting the presence of each solvent atom type in each volume element along the at least one trajectory of the at least one co-solvated simulation system to give the total occupancy over the simulated time in the volume element; g) computer means for using the results in the last step for obtaining free energies of binding to the macromolecule of the different atom types found in the solvent molecules; h) computer means for decoupling the contribution of each atom type in the co-solvent molecule from the rest of atom types of the co-solvent molecule to obtain corrected free energies of binding of the atom type in each volume element by carrying out a step selected from the group consisting of: h1) in step f) redistributing the count units amongst the atom types of the co-solvent molecule so that the count assigned to the atom type is directly proportional to its binding free energy contribution; and h2) in step g) subtracting the binding free energy due to all the other atom types present in the co-solvent molecule from the uncorrected binding free energy of the atom type; i) computer means for gathering all the corrected free energies of binding obtained in step h) for all the atom types to build a corrected scoring function; and j) computer means for based on docking, calculating the binding free energy of the molecule binding to the macromolecular receptor using a scoring function, and rescoring the docked binding molecule using the corrected scoring function obtained in step i).

Further, it is also considered part of the invention a method of computational chemistry for calculating binding free energies to a macromolecular receptor using data derived from classical simulations, which are carried out in a mixed explicit solvent, comprising: a) inputting the 3D structure of a macromolecular receptor in a computer system; b) building at least one simulation system comprising the macromolecular receptor and a mixture of explicit solvents comprising water and at least one organic amphiphilic co-solvent; c) assigning atom types to the atoms in the solvent molecules; d) calculating trajectories of the co-solvated systems; e) partitioning the systems into volume elements; f) counting the presence of each solvent atom type in each volume element along the trajectories to give the total occupancy over the simulated time in the volume element; g) using the results in the last step for obtaining free energies of binding to the macromolecule of the different atom types in the co-solvent molecule in step f) or step g) from the rest of the co-solvent molecule to obtain corrected free energies of binding; and i) using the corrected free energies of binding obtained in step h) to build a scoring function.

Finally, it is also considered a part of the invention a system of computational chemistry for calculating binding free energies to a macromolecular receptor using data derived from classical simulations, which are carried out in a mixed explicit solvent, comprising: a) computer means for inputting the 3D structure of a macromolecular receptor in a computer system; b) computer means for building at least one simulation system comprising the macromolecular receptor and a mixture of explicit solvents comprising water and at least one organic amphiphilic co-solvent; c) computer means for assigning atom types to the atoms in the solvent molecules; d) computer means for calculating trajectories of the co-solvated systems; e) computer means for partitioning the systems into volume elements; f) computer means for counting the presence of each solvent atom type in each volume element along the trajectories to give the total occupancy over the simulated time in the volume element; g) computer means for using the results of the last step for obtaining free energies of binding to the macromolecule of the different atom types found in the solvent molecules; h) computer means for decoupling the contribution of each atom type in the co-solvent molecule in step f) or step g) from the rest of the co-solvent molecule to obtain corrected free energies of binding; and i) computer means for using the corrected free energies of binding obtained in step h) to build a scoring function.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim, and shall not be construed as limiting the scope of the claim. Furthermore, the present invention covers all possible combinations of particular and preferred embodiments described herein.

EXAMPLES

EXAMPLE 1

In this example, the performance of the method of the invention was compared to the performance of other methods used in the art in a virtual screening context. Virtual screening of libraries can be an attractive alternative to experimental high throughput screening (HTS) for finding hits, which are the starting point for the generation of a clinical candidate.

The comparison was carried out in the following way. Two targets of pharmacological interest were taken as subjects, namely thrombin and HSP90. Thrombin is a serine protease that plays a key role in the blood coagulation cascade and HSP90 is a molecular chaperone that has been linked to cancer. These two targets have been extensively studied structurally, and are widely used in the validation of *in silico* methods.

A set of inhibitors for each of these targets was compiled, and mixed with a set of inactive compounds (decoys) taken from the DUD library (Directory of Useful Decoys - Huang, N., et. al. "Benchmarking set for molecular docking", J.Med.Chem. 2006, vol. 16, pp. 6789-6801). The total number for each case can be found in Table 1:

Table 1. Composition of the Virtual Screening sets used.

|  | Active Compounds | Inactive Compounds |
|---|---|---|
| HSP90 | 34 | 860 |
| THROMBIN | 41 | 2292 |

For the 2 systems, these two sets composed of actives and inactives were docked using the rDock program defining the cavity using one of the binder molecules and using default parameters. 100 docking poses per ligand of the decoys set were generated, and the best scoring pose was chosen. For the set of ligands, the experimental binding modes were used. All the selected conformations were then re-scored using 7 methods:

1. Two scoring functions (named RAW_ETA and RAW_ISO, see below) based on the binding free energies derived from molecular dynamics simulations of thrombin or HSP90 carried out in mixed explicit solvent without the application of any corrections, that is, based on ΔGs derived by applying equation (1).

2. Two scoring functions (named CORR_ETA and CORR_ISO, see below) based on the binding free energies derived from molecular dynamics simulations of thrombin or HSP90 carried out in mixed explicit solvent with the decoupling correction that forms part of the invention. In particular, for this case equation (6) with $\alpha$ values obtained from equation (5) was used for calculation of corrected free energies of binding.

For comparison, Glide, a docking program widely in use in the pharmaceutical industry, was used independently and in parallel to dock the inactive set and to minimize and score the experimental binding mode of the molecules in the active set, using two scoring functions provided with the program, namely SP and XP. Also, the scoring given by rDock was used in the comparison. Therefore, among the 7 methods, there were 4 which were simulation-based (RAW_ETA and RAW_ISO, CORR_ETA and CORR_ISO), and the other 3 were based on docking-scoring (Glide XP, Glide SP, and rDock).

For points 1 and 2 above, i.e. for the simulation based methods, the following systems were built for both targets:

a) Ethanol. A solvent mixture of water+ethanol was used to solvate the receptor.
b) Acetamide. A solvent mixture of water+acetamide was used to solvate the receptor.
c) Ionized. A solvent mixture of water+methylammonium+acetate was used to solvate the receptor.
d) Isopropanol. A solvent mixture of water+isopropanol was used to solvate the receptor.

Three replicas of each condition were prepared for running molecular dynamics simulations. All these systems were run under NPT conditions at 300K, with a time step of 2 fs., for 20 ns each. Harmonic constraints were applied over the heavy atoms of the macromolecule to keep the coordinates static taking the crystal structure positions as reference. Snapshots were recorded every 500 steps.

The scoring functions named RAW_ETA, RAW_ISO, CORR_ETA and CORR_ISO are built with the same procedure described hereafter:

1) Obtain the grids with $\Delta G_{bind}$ for each atom type. The exact formulas applied will vary depending on the particular case (see below). The atom types are obtained from the simulations according to Table 2.
2) Assignation of atom types to each molecule in the set to re-score.
3) For every atom in a given molecule of the set, calculate the corresponding grid position according to the atom coordinate and extract all the free energy values of that point and those 0.5 Angstroms around it. Calculate a final atomic value using a Boltzmann average.
4) Sum up all the atomic contributions to obtain the final score of the molecule.

Table 2. Assignation of atom types to co-solvent molecule atoms.

| Co-solvent | Atom Type | chemical attributes |
|---|---|---|
| Ethanol | OH | Hydrogen bond donor and acceptor |
| | CH3 | Hydrophobic |
| Isopropanol | OH | Hydrogen bond donor and acceptor |
| | CH3 | Hydrophobic |
| Acetamide | NH2 | Hydrogen bond donor |
| | O | Hydrogen bond acceptor |
| Methylammonium | NH3+ | Cation |
| Acetate | O- | Anion |

The scoring function termed RAW_ETA, was obtained by applying equation (1) to the data derived from simulations a), b) and c), that is, the uncorrected ΔGs of binding for all atom types were derived from the distributions of water, ethanol, acetamide, methylammonium and acetate solvating the two macromolecular receptors. The scoring function termed RAW_ISO was obtained by applying equation (1) to the data derived from simulations b), c) and d), that is, the uncorrected ΔGs of binding for all atom types were derived from the distributions of water, acetamide and methylammonium, acetate and isopropanol solvating the two macromolecular receptors.

The scoring function termed CORR_ETA, was obtained by applying equation (6) with $\alpha$ values obtained from

equation (5) to the data derived from simulations a), b) and c), that is, the corrected ΔGs of binding for all atom types were derived from the distributions of water, ethanol, acetamide, methylammonium and acetate solvating the two macromolecular receptors. The scoring function termed CORR_ISO was obtained by applying equation (6) with α values obtained from equation (5) to the data derived from simulations b), c) and d), that is, the corrected ΔGs of binding for all atom types were derived from the distributions of water, acetamide and methylammonium, acetate and isopropanol solvating the two macromolecular receptors.

Thus, RAW_ETA and RAW_ISO were obtained by applying the formula found in the prior art, while CORR_ETA and CORR_ISO were obtained by applying the method of the invention . By having two different scoring functions for each case, it was assured that the differences in performance found between corrected and uncorrected scores did not depend on a particular combination of organic co-solvents used in the simulations. By using three different scoring functions implemented in two different programs we compare the method of the invention with widely used methods in structure-based drug discovery.

In order to check which of the methods listed above more reliably selected the active compounds as top scorers of each of the two libraries in a virtual screening calculation, a set of different metrics was used. The scoring method that is best at prioritizing the true binders in the top % of the database screened is taken to be more accurate, and therefore more reliable in a virtual screening campaign in search of hits for a biological target. The metrics used for testing all the different methods was based on Enrichment Factors, which are often used by the skilled person. In particular, the results are presented in terms of the following parameters: *F* is the Fraction of the library selected at the top of the ranked library; *C* is the Completeness, or the fraction of active compounds in *F* (both in percentage); *EF* is the Enrichment Factor. The corresponding formulae are:

$$F = \frac{n}{N} \times 100 \qquad (9)$$

$$C = \frac{a}{A} \times 100 \qquad (10)$$

$$EF = \frac{a/n}{A/N} = \frac{C}{F} \qquad (11)$$

Where N is the total size of the library; n the number of compounds selected after screening; A the total number of active compounds; and a the number of true binders in the selection. Enrichment Factors were calculated for the two targets for the top 5, 10 and 20 % of the compound library. The results of this analysis can be found in Table 3 below:

| THROMBIN | EF5 | EF10 | EF20 |
|---|---|---|---|
| RAW_ETA | 6.85 | 4.40 | 2.93 |
| RAW_ISO | 8.24 | 4.89 | 3.05 |
| CORR_ETA | 11.15 **(+62.8%)** | 6.36 **(+44.5%)** | 3.88 **(+32.4%)** |
| CORR_ISO | 10.67 **(+29.5%)** | 6.57 **(+34.4%)** | 3.64 **(+19.3%)** |
| RDOCK | 7.80 *[-30.0%]* | 4.88 *[-23.3%]* | 3.05 *[-21.4%]* |
| GLIDE_SP | 3.41 *[-69.4%]* | 4.63 *[-27.2%]* | 2.93 *[-24.5%]* |
| GLIDE_XP | 15.61 *[+40.0%]* | 8.05 *[+26.6%]* | 4.27 *[+10.1%]* |

| HSP90 | EF5 | EF10 | EF20 |
|---|---|---|---|
| RAW_ETA | 8.82 | 5.59 | 3.24 |
| RAW_ISO | 9.41 | 5.29 | 3.82 |
| CORR_ETA | 9.41 **(+6.7%)** | 6.39 **(+14.3%)** | 4.12 **(+27.2%)** |
| CORR_ISO | 11.76 **(+25.0%)** | 7.76 **(+46.7%)** | 4.51 **(+18.1%)** |
| RDOCK | 0.00 *[-100%]* | 0.00 *[-100%]* | 0.59 *[-85.7%]* |
| GLIDE_SP | 0.00 *[-100%]* | 0.00 *[-100%]* | 0.15 *[-96.4%]* |
| GLIDE_XP | 1.76 *[-81.3%]* | 1.76 *[-72.5%]* | 1.47 *[-64.3%]* |

Table 3. The results of the enrichment factors (EF) for the top 5, 10 and 20% of the database screened are found for the two targets used in this example. Values in **bold** correspond to % improvement provided by the decoupling correction (CORR_ETA and CORR_ISO over raw scores RAW_ETA and RAW_ISO, respectively). Values in *italics* represent the percentage change in performance of 3 different docking methods relative to CORR_ETA over standard docking.

Inspection of Table 3 clearly shows that CORR_ETA and CORR_ISO, that is, the methods of the invention, clearly outperform RAW_ETA and RAW_ISO, the methods based on the uncorrected ΔG derived from simulation (the improvement found for both targets is between 6.7 and 62.8%). It is also interesting to notice that both corrected methods perform the best, although the actual combination of organic co-solvents used for each is different. It is also remarkable that the method of the invention clearly outperforms docking-scoring methods such as rDock, also for both targets. The only exception found is the performance of Glide XP for thrombin, which is better than that found for CORR_ETA and CORR_ISO (EF5 is 15.6 versus 11.15 and 10.67, respectively). However, it must be clearly pointed out that thrombin was in the training set of macromolecules used to calibrate the Glide XP scoring function - that is, the parameters used to build Glide XP were optimized to give good results on a set of macromolecules among which thrombin was found. The performance of Glide XP is lower for the second system tested, which is much closer to a real drug discovery scenario where the target under study has not been used to build any of the parameterized models.

Thus, it was surprisingly found that the application of the method of the invention performs better at prioritizing true hits in a *in silico* virtual screening campaign for two different targets of therapeutic interest as compared to the prior art methods. Interestingly, the performance of the method holds with two scoring functions based on two different sets of simulations using different combinations of organic co-solvents. Another point to remark is the total absence of any parameterization in the scoring functions proposed in the method herein disclosed. Also worthy of note is the fact that water molecules are essential to mediate the interaction between HSP90 and its ligands; when such water molecules are not considered, as often done by standard docking protocols, virtual screening results are significantly downgraded, as observed here for all three docking methods. Previous empirical knowledge about the role of water molecules is often necessary to obtain more reasonable enrichment factors. In the case of mixed solvent simulations, such knowledge is not necessary, as the water positions are freely exchanged with the organic solvent molecules and the corrected free energy values implicitly account for the effect of preferential hydration sites. As demonstrated in the HSP90 example, this property confers a major advantage to the method.

EXAMPLE 2

In this example, the performance of the method of the invention was compared to the performance of other methods used in the art for the optimization of the biological activity of a chemical series, which binds to a target of pharmacological interest.

The macromolecule analyzed was thrombin. This is a serine protease that plays a key role in the blood coagulation cascade, and therefore has extensively been studied by the pharmaceutical industry as a target linked to diseases associated to blood clotting. Many inhibitors of this enzyme have been disclosed and patented, and a body of research data is available that can be used in the validation of *in silico* tools. One of the most interesting papers disclosed on the binding of thrombin inhibitors to their target is Baum B, et. al. "Non-additivity of functional group contributions in protein-ligand binding: A comparative study by crystallography and isothermal titration calorimetry" J. Mol. Biol. 2010, vol. 397, pp. 1042-1054. In this reference, a set of thrombin inhibitors are disclosed, together with the thermodynamic data associated to their binding to thrombin. Because of the richness of the data disclosed, this set of inhibitors was considered as ideal to test the invention disclosed herewith (Table 4 depicts the structures of the inhibitors used). They span a few orders of magnitude in binding affinity, and can thus be taken to be representative of a series of molecules in a hit to lead campaign, that is, in a situation where a hit is known for a biological target, and its activity (potency) must be optimized in order to increase its therapeutic effect. It must be borne in mind that it is a specially challenging set due to the fact that their binding exhibits non-additive behaviour, that is, two ligand fragments linked together result in a ligand with binding affinity greater than the sum of its parts.

Table 4. Set of thrombin inhibitors that were used for comparison between the standard methods used in the art and the method of the present invention.

The goal was to compare the binding free energies estimated with some of the *in silico* methods widely in use in the art and also the binding free energies obtained with the invention presently disclosed, with the experimental binding free energies found in the abovementioned reference, which were obtained by Isothermal Titration Calorimetry (ITC). In principle, the closer the *in silico* estimation to the experimental results, the more reliable the method is. Thus, the free energy estimations that give closer values to the experimental ones should be taken as the most accurate. Importantly, both the absolute and relative free energies for each of the methods used were monitored.

The *in silico* methods evaluated were:

1. The results of the binding free energy estimation based on docking and scoring calculations carried out with a proprietary docking software, rDock.
2. The results of the binding free energy estimation based on docking and scoring with a widely used software in the pharmaceutical industry, Glide from Schrodinger Inc.
3. The results of the binding free energy estimation based on molecular dynamics simulations of thrombin carried out in mixed explicit solvent without the application of any corrections.
4. The results of the binding free energy estimation based on molecular dynamics simulations of thrombin carried out in mixed explicit solvent with the decoupling correction that forms part of the present invention.

The calculations were carried out the following way. For the docking and scoring calculations (methods 1 and 2), the receptor (thrombin) was taken as a rigid particle, and the crystal structures of the set of inhibitors were aligned over the receptor and scored without modification of the atomic positions (no docking was performed).

For the molecular dynamics based methods (methods 3 and 4), the following procedure was used. Thrombin was solvated in 3 different solvent mixtures separately. In one simulation, the enzyme was modelled in a mixture of water and ethanol. In a second simulation, the enzyme was modelled in a mixture of water and acetamide. In a third simulation, the enzyme was modelled in a mixture of water plus acetate and methylammonium. For inspection of the chemical structures of the solvents used in this example, see Table 5 found below. Three replicas for the 3 systems were modelled. All the replicas were first equilibrated keeping the protein static by applying a harmonic constraint force on the heavy atoms. Then for each of the systems a molecular dynamics trajectory was calculated for 20 ns. Under the NPT ensemble, 2fs. timestep and recording snapshots every 500 steps. Protein was kept rigid applying same constraints as in the equilibration steps.

Table 5. Chemical structures of the organic co-solvents used to solvate thrombin to build the 3 systems used in the example. The three simulations used had a mixture of water+ethanol (right), water+acetamide (middle) and water+methylammonium+acetate (left) (the ionization states used for the co-solvents were the ones depicted).

In this case, the partitioning of the systems used was based on a grid. Once the MD trajectories for the three systems were obtained, the snapshots taken from each of the simulations were superimposed on the same image of the receptor to eliminate rotation and translation, and then the system was immersed in a cubic grid. The spacing of the grid was 0.5 angstroms.

For method 3, the binding free energies for each of the atom types used were calculated via equation (1), which is the current method used in the art. In formula (1), $k_B$ stands for Boltzmann constant, T is the temperature of the system.

Units were converted to kcal/mol by multiplying $k_B$ times Avogadro's number ($N_A$).

For method 4, the binding free energies for each of the atom types used were obtained by applying a correction to method 3. The formula used to carry out the decoupling in this case was (6) with $\alpha$ values obtained from equation (4).

By applying (6) and (4), it is made sure that the binding free energy of the polar head groups found in ethanol, acetamide, methylammonium and acetate derived from the three simulations are decoupled from the binding free energies of the apolar tails found in these organic solvents.

An estimation of the binding free energy of all inhibitors found in Table 4 to thrombin was computed for the four methods, and both the correlation and the slope were obtained in each case. A summary of all the raw data, as well as the statistical measures derived from them can be found in Table 6.

Table 6. Raw (upper part) and statistical data (lower part) obtained by the 4 methods tested to obtain *in silico* free estimates of binding of the set of inhibitors found in Table 4 to thrombin. Exprtl. ΔG holds the experimental binding free energies found in the reference Baum et. al. RDOCK holds the binding free energy estimations with the rDock docking and scoring program.

| | | RDOCK | | GLIDE XP | | UNCORRECTED | | DECOUPLED | |
|---|---|---|---|---|---|---|---|---|---|
| | Exprtl. ΔG | Pred. ΔG | Error | Pred. ΔG | Error | Pred. ΔG | Error | Pred. ΔG | Error |
| **2c** | -7,48 | -19,57 | 12,09 | -6,76 | -0,72 | -12,86 | 5,38 | -4,92 | -2,56 |
| **2e** | -8,39 | -24,04 | 15,65 | -9,21 | 0,82 | -16,90 | 8,51 | -6,60 | -1,79 |
| **2l** | -8,46 | -24,80 | 16,34 | -9,77 | 1,31 | -20,42 | 11,96 | -7,86 | -0,60 |
| **3c** | -7,84 | -25,24 | 17,40 | -11,31 | 3,47 | -18,55 | 10,71 | -6,87 | -0,97 |
| **3e** | -8,94 | -21,69 | 12,75 | -12,05 | 3,11 | -24,69 | 15,75 | -9,62 | 0,68 |
| **3l** | -9,03 | -28,41 | 19,38 | -12,58 | 3,55 | -25,54 | 16,51 | -9,48 | 0,45 |
| **4c** | -9,58 | -25,17 | 15,59 | -9,46 | -0,12 | -19,99 | 10,41 | -7,78 | -1,80 |
| **4e** | -10,25 | -29,68 | 19,43 | -12,42 | 2,17 | -24,54 | 14,29 | -9,69 | -0,56 |
| **4l** | -11,02 | -32,23 | 21,21 | -12,76 | 1,74 | -26,96 | 15,94 | -10,61 | -0,41 |

| RDOCK | | | GLIDE XP | | |
|---|---|---|---|---|---|
| r2 | rho | slope | r2 | rho | slop e |
| 0,68 | 0,75 | 2,86 | 0,44 | 0,73 | 1,19 |

| UNCORRECTED | | | DECOUPLED | | |
|---|---|---|---|---|---|
| r2 | rho | slope | r2 | rho | slop e |
| 0,64 | 0,8 | 3,28 | 0,71 | 0,87 | 1,37 |

GLIDE XP holds the binding free energy estimations with the Glide docking program, and its best scoring function, XP. UNCORRECTED holds the binding free energy estimation based on mixed-MD simulations without the application of any corrections (that is, obtained by formula (1)). DECOUPLED holds the binding free energy estimation based on mixed-MD simulations with the application of the decoupling correction (that is, obtained by formulas (6) and (4)). ΔG units are kcal/mol.

As can be seen, the results of the 4 methods are not comparable. The rDock calculations give absolute free energies of binding which are clearly deviated from the experimental ones, this is reflected in both the raw values (from -19.57 to -32.23 kcal/mol) and the slope found for this dataset (2.86). However, the relative free energies given by this docking and scoring method are quite good, with a correlation ($r^2$) of 0.68. The Glide XP calculations on the other hand, give absolute free energies of binding that are superior to those obtained by rDock, with values ranging from -6.76 to -12.76 kcal/mol, closer the experimental ones (the errors are much smaller). This is also reflected in the slope, which is 1.19. However, the relative free energies within this congeneric series of inhibitors are poorer than those found for rDock, with a correlation of 0.44. This means that for thrombin, rDock is more able to rank order the compounds based on their potency than Glide XP. However, in absolute terms, the values given by Glide XP are closer to the experimental values than those given by rDock.

The Uncorrected values derived from the MD simulations in explicit mixed solvent are also very much deviated from the experimental values in absolute terms, as can be seen by the big error values and the large slope (3.28).

However, the relative estimates are in good agreement with the experimental values, as the correlation is 0.64. This means that the standard method found in the art to extract free energies of binding from mixed solvent simulations is good enough for rank ordering of a set of ligands, but not to extract absolute free energies of binding to a macromolecular receptor. However, the method of the present invention, based on decoupling of the binding free energies extracted from the organic co-solvents is better at both estimating the absolute free energies, and also the relative rank ordering. Inspection of Table 6 (Decoupled) clearly points to the fact that the correction applied results in much more realistic absolute free energies of binding. Thus, the errors are much smaller as compared to the errors found for the uncorrected method. The slope is much closer to 1 (1.37). Moreover, the relative free energies are also superior to the uncorrected method, as in this case, the correlation within this congeneric series is found to be 0.71. Thus, for thrombin and the series of inhibitors found in Table 4, it was seen that the method disclosed herein is superior to two docking and scoring methods widely in use, and to the method of obtaining uncorrected free energies that is found in the prior art.

EXAMPLE 3

In this example, the contours derived from the application of the method of the invention are compared to the contours derived from molecular dynamics simulations without the application of any correction, that is, the corrected free energies obtained by formula (6) with $\alpha$ values obtained from equation (5) are compared to the uncorrected free energies derived from formula (1). The system used to carry out this qualitative comparison was HSP90. A co-crystal structure of this enzyme together with an inhibitor with a known binding mode was used to compare calculated contours with actual protein-ligand interactions. The contours with the highest quality should map correctly the different chemical features found in the inhibitor. That is, it was expected that the contours for a favourable interaction with a hydrophobic atom type (such as a methyl found in isopropanol) would overlap with the hydrophobic parts of the known inhibitor.
As can be seen in FIG.1, the contours derived from the mixed explicit solvent molecular dynamics without any correction for the hydrophobic probe are overlapping mostly with the hydrophobic parts of the inhibitor. However, they also show some degree of overlap with a polar part of the inhibitor (they cover a wide area engulfing the amino group and one of the pyrimidine nitrogen atoms). In contrast, the contours derived from the application of the method of the invention nearly disappear in the mentioned polar area of the inhibitor, and at the same time are slightly expanded in the hydrophobic part. In other words, the corrected free energy grids more accurately distinguish favourable areas for a polar or an apolar probe atom as compared to the uncorrected grids.
Thus, it was concluded that the contours derived from the application of the method of the invention can be more reliably used as a visual guide on the different spots that are found in a binding site of a macromolecule when analyzed by using a computer graphics system, and therefore give more accurate clues on what chemical features should be present in what areas of the active site. This can be a valuable guide in the rational design of inhibitors of a macromolecular target.

REFERENCES CITED IN THE APPLICATION

Hann MM. et. al. "Molecular complexity and its impact on the probability of finding leads for drug discovery" J. Chem. Inf. Comput. Sci. 2001, vol. 41, pp. 856-864.

Orozco M., Luque FJ. "Theoretical methods for the description of the solvent effect in biomolecular systems" Chem. Rev. 2000, vol. 100, pp. 4187-4226.

Young T., et. al. "Dewetting transitions in protein cavities" Proteins 2010, vol. 78, pp. 1856-1869

Young T., et.al. "Motifs for molecular recognition exploiting hydrophobic enclosure in protein-ligand binding" PNAS 2006, vol. 104, pp. 808-813.

WO 2010/129386 A2

Kuntz ID., et.al. "The maximal affinity of ligands" PNAS 1999, vol. 96, pp. 9997-10002.

Fersht AR. "The hydrogen bond in molecular recognition" Trends in Biochemical Science, vol.12, pp. 301-304

Karplus PA. "Hydrophobicity regained" Protein Science 1997, vol.6, pp. 1302-1307

**EP 2 795 498 B1**

Huang, N., et. al. "Benchmarking set for molecular docking", J.Med.Chem. 2006, vol. 16, pp. 6789-6801

Baum B, et. al. "Non-additivity of functional group contributions in protein-ligand binding: A comparative study by crystallography and isothermal titration calorimetry" J. Mol. Biol. 2010, vol. 397, pp. 1042-1054.

**Claims**

1. A method of computational chemistry for calculating binding free energy/ies of a molecule binding to a macromolecular receptor using data derived from classical simulations, which are carried out in a mixed explicit solvent, comprising:

   a) inputting the 3D structure of a macromolecular receptor in a computer system;
   b) building at least one co-solvated simulation system comprising the macromolecular receptor and a mixture of explicit solvents comprising water and at least one organic amphiphilic co-solvent;
   c) assigning atom types to the atoms in the solvent molecules of the at least one co-solvated simulation system of step b);
   d) calculating at least one trajectory of the at least one co-solvated simulation system;
   e) partitioning the at least one co-solvated simulation system into volume elements;
   f) counting the presence of each solvent atom type in each volume element along the at least one trajectory of the at least one co-solvated simulation system to give the total occupancy over the simulated time in the volume element;
   g) using the results in the last step for obtaining free energies of binding to the macromolecule of the different atom types found in the solvent molecules;
   h) decoupling the contribution of each atom type in the co-solvent molecule from the rest of atom types of the co-solvent molecule to obtain corrected free energies of binding of the atom type in each volume element by carrying out a step selected from the group consisting of:

      h1) in step f) redistributing the count units amongst the atom types of the co-solvent molecule so that the count assigned to the atom type is directly proportional to its binding free energy contribution; and
      h2) in step g) subtracting the binding free energy due to all the other atom types present in the co-solvent molecule from the uncorrected binding free energy of the atom type.

   i) gathering all the corrected free energies of binding obtained in step h) for all the atom types to build a corrected scoring function; and
   j) based on docking, calculating the binding free energy of the molecule binding to the macromolecular receptor using the corrected scoring function obtained in step i).

2. A method of computational chemistry for calculating binding free energy/ies of a molecule binding to a macromolecular receptor using data derived from classical simulations, which are carried out in a mixed explicit solvent, comprising:

   a) inputting the 3D structure of a macromolecular receptor in a computer system;
   b) building at least one co-solvated simulation system comprising the macromolecular receptor and a mixture of explicit solvents comprising water and at least one organic amphiphilic co-solvent;
   c) assigning atom types to the atoms in the solvent molecules of the at least one co-solvated simulation system of step b);
   d) calculating at least one trajectory of the at least one co-solvated simulation system;
   e) partitioning the at least one co-solvated simulation system into volume elements;
   f) counting the presence of each solvent atom type in each volume element along the at least one trajectory of the at least one co-solvated simulation system to give the total occupancy over the simulated time in the volume element;
   g) using the results in the last step for obtaining free energies of binding to the macromolecule of the different atom types found in the solvent molecules;
   h) decoupling the contribution of each atom type in the co-solvent molecule from the rest of atom types of the co-solvent molecule to obtain corrected free energies of binding of the atom type in each volume element by carrying out a step selected from the group consisting of:

      h1) in step f) redistributing the count units amongst the atom types of the co-solvent molecule so that the count assigned to the atom type is directly proportional to its binding free energy contribution; and

h2) in step g) subtracting the binding free energy due to all the other atom types present in the co-solvent molecule from the uncorrected binding free energy of the atom type.

i) gathering all the corrected free energies of binding obtained in step h) for all the atom types to build a corrected scoring function; and

j) based on docking, calculating the binding free energy of the molecule binding to the macromolecular receptor using a scoring function, and rescoring the docked binding molecule using the corrected scoring function obtained in step i).

3. The method according to any of the claims 1 to 2, further comprising carrying out a local concentration correction prior to step g), comprising:

3-1) counting the number of contacts that each solvent molecule bearing the atom type present in the volume element makes with the rest of molecules belonging to the same type of solvent;

3-2) running a series of simulations without any solute where there are different concentrations of the organic co-solvent plus water, to derive a relationship between global macroscopic concentration of the organic co-solvent and average number of contacts between the molecules of the organic co-solvent;

3-3) converting the number of contacts calculated in step 3-1) to the real concentration in each volume element by using the correspondence obtained in step 3-2).

4. The method according to any of the claims 1 to 3, further comprising a step k) wherein visualization of the free energy profiles of each atom type on the surface of the receptor is carried out in a computer graphics system.

5. The method according to any of the claims 1 to 4, wherein the calculation of the at least one trajectory of the at least one co-solvated simulation system is carried out with a molecular dynamics simulation.

6. The method according to any of the claims 1 to 4, wherein the calculation of the at least one trajectory of the at least one co-solvated simulation system is carried out with a MonteCarlo simulation.

7. The method according to any of the claims 1 to 6, wherein the organic amphiphilic co-solvent is selected from the group consisting of a (C1-C8)-alcohol, a (C2-C8)-amide, a (C1-C8)-amine, a (C2-C8)-carboxylic acid, a (C2-C8)-ether, a (C2-C8)-thioether, a (C2-C8)-sulphone, a (C1-C8)-sulphonamide, a 5-7 membered saturated or unsaturated heterocycle containing S, O, or N, acetone, acetonitrile, urea, and a mixture of methylammonium and acetate, a (C1-C8)-alcohol substituted by one or more halogens, a (C2-C8)-amide substituted by one or more halogens, a (C1-C8)-amine substituted by one or more halogens, a (C2-C8)-carboxylic acid substituted by one or more halogens, a (C2-C8)-ether substituted by one or more halogens, a (C2-C8)-thioether substituted by one or more halogens, a (C2-C8)-sulphone substituted by one or more halogens, a (C1-C8)-sulphonamide substituted by one or more halogens, a 5-7 membered saturated or unsaturated heterocycle containing S, O, or N substituted by one or more halogens..

8. The method according to claim 7, wherein the organic co-solvent is selected from the group consisting of ethanol, isopropanol, acetamide, pyridine, pyrimidine, piperazine, morpholine, pyrrole, oxazole, pyrazole, pyrrolidone, acetone, acetonitrile, trifluoroethanol, vinylamine, dimethylether, urea, and a mixture of methylammonium and acetate.

9. The method according to any of the claims 1 to 8, where step d) is performed with the application of Cartesian restraints on the receptor.

10. The method according to any of the claims 1 to 9, wherein the macromolecular receptor is a protein or a glycoprotein.

11. The method according to any of the claims 1 to 9, wherein the macromolecular receptor is a nucleic acid.

12. A computer program product comprising program instructions for causing a computer to perform the method of computational chemistry for calculating binding free energy/ies of a molecule binding to a macromolecular receptor as defined in any of the claims 1 to 11.

13. The computer program product according to claim 12, embodied on a storage medium.

**14.** The computer program product according to claim 12, carried on a carrier signal.

**15.** A system of computational chemistry for calculating binding free energy/ies of a molecule binding to a macromolecular receptor using data derived from classical simulations, which are carried out in a mixed explicit solvent, comprising:

a) computer means for inputting the 3D structure of a macromolecular receptor in a computer system;

b) computer means for building at least one co-solvated simulation system comprising the macromolecular receptor and a mixture of explicit solvents comprising water and at least one organic amphiphilic co-solvent;

c) computer means for assigning atom types to the atoms in the solvent molecules of the at least one co-solvated simulation system of step b);

d) computer means for calculating at least one trajectory of the at least one co-solvated simulation system;

e) computer means for partitioning the at least one co-solvated simulation system into volume elements;

f) computer means for counting the presence of each solvent atom type in each volume element along the at least one trajectory of the at least one co-solvated simulation system to give the total occupancy over the simulated time in the volume element;

g) computer means for using the results in the last step for obtaining free energies of binding to the macromolecule of the different atom types found in the solvent molecules;

h) computer means for decoupling the contribution of each atom type in the co-solvent molecule from the rest of atom types of the co-solvent molecule to obtain corrected free energies of binding of the atom type in each volume element by carrying out a step selected from the group consisting of:

h1) in step f) redistributing the count units amongst the atom types of the co-solvent molecule so that the count assigned to the atom type is directly proportional to its binding free energy contribution; and

h2) in step g) subtracting the binding free energy due to all the other atom types present in the co-solvent molecule from the uncorrected binding free energy of the atom type.

i) computer means for gathering all the corrected free energies of binding obtained in step h) for all the atom types to build a corrected scoring function; and

j) computer means for based on docking, calculating the binding free energy of the molecule binding to the macromolecular receptor using the corrected scoring function obtained in step i).

**Patentansprüche**

**1.** Ein Verfahren der computergestützten Chemie zur Berechnung der freien Bindungsenergie(n) eines sich an einen makromolekularen Rezeptor bindenden Moleküls unter Verwendung von aus klassischen Simulationen erhaltenen Daten, welche in einem gemischten expliziten Lösungsmittel durchgeführt werden, umfassend:

a) eingeben der 3D-Struktur eines makromolekularen Rezeptors in ein Computersystem;

b) bilden von mindestens einem cosolvatisierten Simulationssystem umfassend den makromolekularen Rezeptor und eine Mischung aus expliziten Lösungsmitteln umfassend Wasser und mindestens ein organisches amphiphiles Colösungsmittel;

c) zuordnen von Atomtypen den Atomen der Lösungsmittelmoleküle des mindestens einen cosolvatisierten Simulationssystems des Schritts b);

d) berechnen von mindestens einer Trajektorie des mindestens einen cosolvatisierten Simulationssystems;

e) teilen des mindestens einen cosolvatisierten Simulationssystems in Volumenelemente;

f) zählen der Anwesenheit von jedem Lösungsmittelatomtypen in jedem Volumenelement zusammen mit der mindestens einen Trajektorie des mindestens einen cosolvatisierten Simulationssystems, um die gesamte Belegung während der simulierten Zeit im Volumenelement zu erhalten;

g) verwenden der Ergebnisse des letzten Schritts, um freie Energie der Bindung an das Makromolekül durch die verschiedenen in den Lösungsmittelmolekülen festgestellten Atomtypen zu erhalten;

h) entkoppeln des Beitrags von jedem Atomtypen des Colösungsmittelmoleküls von den restlichen Atomtypen des Colösungsmittelmoleküls, um berichtigte freie Energien der Bindung des Atomtypen in jedem Volumenelement durch die Durchführung von einem aus der folgenden Gruppe ausgewählten Schritt zu erhalten:

h1) umverteilen, im Schritt f), der Zählmengeneinheiten unter den Atomtypen des Colösungsmittelmoleküls, so dass die dem Atomtypen zugeordnete Zählmenge direkt proportional zu seinem Beitrag auf die freie Energie ist;
und
h2) subtrahieren, im Schritt g), der freien Bindungsenergie, die auf alle anderen im Colösungsmittelmolekül anwesenden Atomtypen zurückzuführen ist von der nicht berichtigten freien Bindungsenergie des Atomtypen.

i) ansammeln von allen berichtigten freien Bindungsenergien, die im Schritt h) für alle Atomtypen erhalten worden sind, um eine berichtigte Bewertungsfunktion zu erstellen; und
j) berechnen, beruhend auf *Docking,* der freien Bindungsenergie des sich an den makromolekularen Rezeptor bindenden Moleküls mit Hilfe von der im Schritt i) erhaltenen berichtigten Bewertungsfunktion.

2. Ein Verfahren der computergestützten Chemie zur Berechnung der freien Bindungsenergie(n) eines sich an einen makromolekularen Rezeptor bindenden Moleküls unter Verwendung von aus klassischen Simulationen erhaltenen Daten, welche in einem gemischten expliziten Lösungsmittel durchgeführt werden, umfassend:

a) eingeben der 3D-Struktur eines makromolekularen Rezeptors in ein Computersystem;
b) bilden von mindestens einem cosolvatisierten Simulationssystem umfassend den makromolekularen Rezeptor und eine Mischung aus expliziten Lösungsmitteln umfassend Wasser und mindestens ein organisches amphiphiles Colösungsmittel;
c) zuordnen von Atomtypen den Atomen der Lösungsmittelmoleküle des mindestens einen cosolvatisierten Simulationssystems des Schritts b);
d) berechnen von mindestens einer Trajektorie des mindestens einen cosolvatisierten Simulationssystems;
e) teilen des mindestens einen cosolvatisierten Simulationssystems in Volumenelemente;
f) zählen der Anwesenheit von jedem Lösungsmittelatomtypen in jedem Volumenelement zusammen mit der mindestens einen Trajektorie des mindestens einen cosolvatisierten Simulationssystems, um die gesamte Belegung während der simulierten Zeit im Volumenelement zu erhalten;
g) verwenden der Ergebnisse des letzten Schritts, um freie Energie der Bindung an das Makromolekül durch die verschiedenen in den Lösungsmittelmolekülen festgestellten Atomtypen zu erhalten;
h) entkoppeln des Beitrags von jedem Atomtypen des Colösungsmittelmoleküls von den restlichen Atomtypen des Colösungsmittelmoleküls, um berichtigte freie Energien der Bindung des Atomtypen in jedem Volumenelement durch die Durchführung von einem aus der folgenden Gruppe ausgewählten Schritt zu erhalten:

h1) umverteilen, im Schritt f), der Zählmengeneinheiten unter den Atomtypen des Colösungsmittelmoleküls, so dass die dem Atomtypen zugeordnete Zählmenge direkt proportional zu seinem Beitrag auf die freie Energie ist;
und
h2) subtrahieren, im Schritt g), der freien Bindungsenergie, die auf alle anderen im Colösungsmittelmolekül anwesenden Atomtypen zurückzuführen ist von der nicht berichtigten freien Bindungsenergie des Atomtypen.

i) ansammeln von allen berichtigten freien Bindungsenergien, die im Schritt h) für alle Atomtypen erhalten worden sind, um eine berichtigte Bewertungsfunktion zu erstellen; und
j) berechnen, beruhend auf *Docking,* der freien Bindungsenergie des sich an den makromolekularen Rezeptor bindenden Moleküls mit Hilfe von einer Bewertungsfunktion, und durchführen von einer neuen Bewertung des bindenden Moleküls, für das das *Docking* eingesetzt worden ist, mit Hilfe von der im Schritt i) erhaltenen berichtigten Bewertungsfunktion.

3. Das Verfahren nach einem der Ansprüche 1 oder 2, weiterhin umfassend das Durchführen von einer örtlichen Konzentrationsberichtigung vordem Schritt g), umfassend:

3-1) zählen der Anzahl von Kontakten, die jedes Lösungsmittelmolekül, welches den im Volumenelement anwesenden Atomtypen trägt, mit den restlichen Molekülen herstellt, die zum selben Lösungsmitteltypen gehören;
3-2) durchführen von einer Reihe von Simulationen, ohne jede gelöste Substanz, wobei verschiedene Konzentrationen des organischen Colösungsmittels neben Wasser vorhanden sind, um einen Zusammenhang zwischen der gesamten makroskopischen Konzentration des organischen Colösungsmittels mit der durchschnittlichen Anzahl von Kontakten zwischen den Molekülen des organischen Colösungsmittels abzuleiten;
3-3) umsetzen der im Schritt 3-1) berechneten Anzahl von Kontakten in tatsächliche Konzentration in jedem Volumenelement mit Hilfe von dem im Schritt 3-2) gefundenen Zusammenhang.

**4.** Das Verfahren nach einem der Ansprüche 1 bis 3, weiterhin umfassend einen Schritt k), wobei die Visualisierung der Profile der freien Energie für jeden Atomtypen auf der Rezeptoroberfläche in einem Computergraphiksystem durchgeführt wird.

**5.** Das Verfahren nach einem der Ansprüche 1 bis 4, wobei die Berechnung von der mindestens einen Trajektorie von dem mindestens einen cosolvatisierten Simulationssystem mittels einer auf Molekulardynamik beruhenden Simulation durchgeführt wird.

**6.** Das Verfahren nach einem der Ansprüche 1 bis 4, wobei die Berechnung von der mindestens einen Trajektorie von dem mindestens einen cosolvatisierten Simulationssystem mittels einer auf Monte-Carlo beruhenden Simulation durchgeführt wird.

**7.** Das Verfahren nach einem der Ansprüche 1 bis 6, wobei das organische amphiphile Colösungsmittel aus der Gruppe bestehend aus einem (C1-C8)-Alkohol, einem (C2-C8)-Amid, einem (C1-C8)-Amin, einer (C2-C8)-Carbonsäure, einem (C2-C8)-Ether, einem (C2-C8)-Thioether, einem (C2-C8)-Sulphon, einem (C1-C8)- Sulphonamid, einem 5-7 gliedrigen gesättigten oder ungesättigten Heterozyklus enthaltend S, O, oder N, Aceton, Acetonitril, Urea, und einer Mischung aus Methylammonium und Acetat, einem durch ein oder mehr Halogene substituierten (C1-C8)-Alkohol, einem durch ein oder mehr Halogene substituierten (C2-C8)-Amid, einem durch ein oder mehr Halogene substituierten (C1-C8)- Amin, einer durch ein oder mehr Halogene substituierten (C2-C8)-Carbonsäure, einem durch ein oder mehr Halogene substituierten (C2-C8)-Ether, einem durch ein oder mehr Halogene substituierten (C2-C8)-Thioether, einem durch ein oder mehr Halogene substituierten (C2-C8)-Sulphon, einem durch ein oder mehr Halogene substituierten (C1-C8)- Sulphonamid, einem 5-7 gliedrigen gesättigten oder ungesättigten Heterozyklus enthaltend S, O, oder N, der durch ein oder mehr Halogene substituiert ist.

**8.** Das Verfahren nach Anspruch 7, wobei das organische Colösungsmittel ausgewählt aus der Gruppe bestehend aus Ethanol, Isopropanol, Acetamid, Pyridin, Pyrimidin, Piperazin, Morpholin, Pyrrol, Oxazol, Pyrazol, Pyrrolidon, Aceton, Acetonitril, Trifluoroethanol, Vinylamin, Dimethylether, Urea, und einer Mischung aus Methylammonium und Acetat ist.

**9.** Das Verfahren nach einem der Ansprüche 1 bis 8, wobei der Schritt d) mit der Anwendung von kartesianischen Einschränkungen auf dem Rezeptor durchgeführt wird.

**10.** Das Verfahren nach einem der Ansprüche 1 bis 9, wobei der makromolekulare Rezeptor ein Protein oder ein Glykoprotein ist.

**11.** Das Verfahren nach einem der Ansprüche 1 bis 9, wobei der makromolekulare Rezeptor eine Nukleinsäure ist.

**12.** Ein Computerprogrammprodukt umfassend Programmanweisungen zur Veranlassung der Durchführung des Verfahrens der computergestützten Chemie zur Berechnung der freien Bindungsenergie(n) eines sich an einen makromolekularen Rezeptor bindenden Moleküls wie in einem der Ansprüche 1 bis 11 definiert durch einen Computer.

**13.** Das Computerprogrammprodukt nach Anspruch 12, das in einem Speichermedium enthalten ist.

**14.** Das Computerprogrammprodukt nach Anspruch 12, das in einem Trägersignal getragen ist.

**15.** Ein System der computergestützten Chemie zur Berechnung der freien Bindungsenergie(n) eines sich an einen makromolekularen Rezeptor bindenden Moleküls unter Verwendung von aus klassischen Simulationen erhaltenen Daten, welche in einem gemischten expliziten Lösungsmittel durchgeführt werden, umfassend:

   a) ein Computermittel zur Eingabe der 3D-Struktur eines makromolekularen Rezeptors in ein Computersystem;
   b) ein Computermittel zur Bildung von mindestens einem cosolvatisierten Simulationssystem umfassend den makromolekularen Rezeptor und eine Mischung aus expliziten Lösungsmitteln umfassend Wasser und mindestens ein organisches amphiphiles Colösungsmittel;
   c) ein Computermittel zur Zuordnung von Atomtypen den Atomen der Lösungsmittelmoleküle des mindestens einen cosolvatisierten Simulationssystem des Schritts b);
   d) ein Computermittel zur Berechnung von mindestens einer Trajektorie des mindestens einen cosolvatisierten Simulationssystems;
   e) ein Computermittel zur Teilung des mindestens einen cosolvatisierten Simulationssystems in Volumenelemente;

f) ein Computermittel zur Zählung der Anwesenheit von jedem Lösungsmittelatomtypen in jedem Volumenelement zusammen mit der mindestens einen Trajektorie des mindestens einen cosolvatisierten Simulationssystems, um die gesamte Belegung während der simulierten Zeit im Volumenelement zu erhalten;

g) ein Computermittel zur Verwendung der Ergebnisse des letzten Schritts, um freie Energien der Bindung am Makromolekül durch die verschiedenen in den Lösungsmittelmolekülen festgestellten Atomtypen zu erhalten;

h) ein Computermittel zur Entkopplung des Beitrags von jedem Atomtypen des Colösungsmittelmoleküls von den restlichen Atomtypen im Colösungsmittelmolekül, um berichtigte freie Energien der Bindung des Atomtypen in jedem Volumenelement durch die Durchführung von einem aus der folgenden Gruppe ausgewählten Schritt zu erhalten:

h1) umverteilen, im Schritt f), der Zählmengeneinheiten unter den Atomtypen des Colösungsmittelmoleküls, so dass die dem Atomtypen zugeordnete Zählmenge direkt proportional zu seinem Beitrag auf die freie Energie ist;

und

h2) subtrahieren, im Schritt g), der freien Bindungsenergie, die auf alle anderen im Colösungsmittelmolekül anwesenden Atomtypen zurückzuführen ist von der nicht berichtigten freien Bindungsenergie des Atomtypen.

i) ein Computermittel zur Ansammlung von allen berichtigten freien Bindungsenergien, die im Schritt h) für alle Atomtypen erhalten worden sind, um eine berichtigte Bewertungsfunktion zu erstellen; und

j) ein Computermittel zur Berechnung, beruhend auf *Docking,* der freien Bindungsenergie des sich an den makromolekularen Rezeptor bindenden Moleküls mit Hilfe von der im Schritt i) erhaltenen berichtigten Bewertungsfunktion.

## Revendications

1. Un procédé de chimie numérique pour calculer le(s) énergie(s) libre(s) de liaison d'une molécule se liant à un récepteur macromoléculaire en utilisant des données obtenues par des simulations classiques, qui sont effectuées dans un solvant explicite mélangé, comprenant :

a) introduire la structure 3D d'un récepteur macromoléculaire dans un système informatique ;

b) établir au moins un système de simulation co-solvaté comprenant le récepteur macromoléculaire et un mélange de solvants explicites comprenant de l'eau et au moins un co-solvant amphiphile organique ;

c) affecter des types d'atome aux atomes des molécules de solvant de l'au moins un système de simulation co-solvaté de l'étape b) ;

d) calculer au moins une trajectoire de l'au moins un système de simulation co-solvaté ;

e) partager l'au moins un système de simulation co-solvaté dans des éléments de volume ;

f) compter la présence de chaque type d'atome de solvant dans chaque élément de volume le long de l'au moins une trajectoire de l'au moins un système de simulation co-solvaté pour donner le taux de remplissage total au cours de la période de simulation dans l'élément de volume ;

g) utiliser les résultats de la dernière étape pour obtenir des énergies libres de liaison à la macromolécule des types d'atome différents trouvés dans les molécules de solvant ;

h) découpler la contribution de chaque type d'atome de la molécule de co-solvant du reste de types d'atome de la molécule de co-solvant pour obtenir les énergies libres de liaison corrigées du type d'atome dans chaque élément de volume en effectuant une étape choisie dans le groupe constitué de :

h1) redistribuer, dans l'étape f), les unités de comptage parmi les types d'atome de la molécule de co-solvant de manière que le comptage affecté au type d'atome soit directement proportionnel à sa contribution à l'énergie libre de liaison ;

et

h2) soustraire, dans l'étape g), l'énergie libre de liaison due à tous les autres types d'atome présents dans la molécule de co-solvant de l'énergie libre de liaison non corrigée du type d'atome.

i) recueillir toutes les énergies libres de liaison corrigées obtenues dans l'étape h) pour tous les types d'atome afin d'établir une fonction de score corrigée ; et

j) sur la base de la méthode d'amarrage, calculer l'énergie libre de liaison de la molécule se liant au récepteur macromoléculaire en utilisant la fonction de score corrigée obtenue dans l'étape i).

2. Un procédé de chimie numérique pour calculer le(s) énergie(s) libre(s) de liaison d'une molécule se liant à un récepteur macromoléculaire en utilisant des données obtenues par des simulations classiques, qui sont effectuées dans un solvant explicite mélangé, comprenant :

a) introduire la structure 3D d'un récepteur macromoléculaire dans un système informatique ;
b) établir au moins un système de simulation co-solvaté comprenant le récepteur macromoléculaire et un mélange de solvants explicites comprenant de l'eau et au moins un co-solvant amphiphile organique ;
c) affecter des types d'atome aux atomes des molécules de solvant de l'au moins un système de simulation co-solvaté de l'étape b) ;
d) calculer au moins une trajectoire de l'au moins un système de simulation co-solvaté ;
e) partager l'au moins un système de simulation co-solvaté dans des éléments de volume ;
f) compter la présence de chaque type d'atome de solvant dans chaque élément de volume le long de l'au moins une trajectoire de l'au moins un système de simulation co-solvaté pour donner le taux de remplissage total au cours de la période de simulation dans l'élément de volume ;
g) utiliser les résultats de la dernière étape pour obtenir des énergies libres de liaison à la macromolécule des types d'atome différents trouvés dans les molécules de solvant ;
h) découpler la contribution de chaque type d'atome de la molécule de co-solvant du reste des types d'atome de la molécule de co-solvant pour obtenir les énergies libres de liaison corrigées du type d'atome dans chaque élément de volume en effectuant une étape choisie dans le groupe constitué de :

h1) redistribuer, dans l'étape f), les unités de comptage parmi les types d'atome de la molécule de co-solvant de manière que le comptage affecté au type d'atome soit directement proportionnel à sa contribution à l'énergie libre de liaison ;
et
h2) soustraire, dans l'étape g), l'énergie libre de liaison due à tous les autres types d'atome présents dans la molécule de co-solvant de l'énergie libre de liaison non corrigée du type d'atome.

i) recueillir toutes les énergies libres de liaison corrigées obtenues dans l'étape h) pour tous les types d'atome afin d'établir une fonction de score corrigée ; et
j) sur la base de la méthode d'amarrage, calculer l'énergie libre de liaison de la molécule se liant au récepteur macromoléculaire en utilisant une fonction de score, et obtenir de nouveau des scores pour la molécule de liaison soumise à amarrage en utilisant la fonction de score corrigée obtenue dans l'étape i).

3. Le procédé selon l'une quelconque des revendications 1 à 2, comprenant en outre la réalisation d'une correction de la concentration locale avant de l'étape g), comprenant :

3-1) compter la quantité de contacts faits par chaque molécule de solvant portant le type d'atome présent dans l'élément de volume avec le reste de molécules appartenant au même type de solvant ;
3-2) effectuer une série de simulations sans aucun soluté, ou il y a des concentrations différentes du co-solvant organique et de l'eau, pour obtenir une relation entre la concentration macroscopique globale du co-solvant organique et la quantité moyenne de contacts entre les molécules du co-solvant organique;
3-3) convertir la quantité de contacts calculée dans l'étape 3-1) en la concentration réelle de chaque élément de volume en utilisant la correspondance obtenue dans l'étape 3-2).

4. Le procédé selon l'une quelconque des revendications 1 à 3, comprenant en outre une étape k) dans laquelle la visualisation des profils d'énergie libre de chaque type d'atome sur la surface du récepteur est effectuée dans un système d'infographie.

5. Le procédé selon l'une quelconque des revendications 1 à 4, dans lequel le calcul de l'au moins une trajectoire de l'au moins un système de simulation co-solvaté est effectué avec une simulation de dynamique moléculaire.

6. Le procédé selon l'une quelconque des revendications 1 à 4, dans lequel le calcul de l'au moins une trajectoire de l'au moins un système de simulation co-solvaté est effectuée avec une simulation de Monte-Carlo.

7. Le procédé selon l'une quelconque des revendications 1 à 6, dans lequel le co-solvant amphiphile organique est choisi dans le groupe constitué d'un alcool en (C1-C8), une amide en (C2-C8), une amine en (C1-C8), un acide carboxylique en (C2-C8), un éther en (C2-C8), un tioéther en (C2-C8), une sulfone en (C2-C8), une sulfonamide en (C1-C8), un hétérocycle saturé ou insaturé à 5-7 chaînons contenant du S, O, ou N, acétone, acétonitrile, urée, et un mélange de méthylammonium et acétate, un alcool en (C1-C8) substitué par un ou plusieurs halogènes, une amide en (C2-C8) substituée par un ou plusieurs halogènes, une amine en (C1-C8) substituée par un ou plusieurs halogènes, un acide carboxylique (C2-C8) substitué par un ou plusieurs halogènes, un éther (C2-C8) substitué par un ou plusieurs halogènes, un tioéther en (C2-C8) substitué par un ou plusieurs halogènes, une sulfone en (C2-C8) substituée par un ou plusieurs halogènes, une sulfonamide en (C1-C8) substituée par un ou plusieurs halogènes, un hétérocycle saturé o insaturé à 5-7 chaînons contenant du S, O, ou N substitué par un ou plusieurs halogènes.

8. Le procédé selon la revendication 7, dans lequel le co-solvant organique est choisi dans le groupe constitué d'éthanol, isopropanol, acétamide, pyridine, pyrimidine, pipérazine, morpholine, pyrrol, oxazol, pyrazol, pyrrolidone, acétone, acétonitrile, trifluoroéthanol, vinylamine, diméthyléther, urée, et un mélange de méthylammonium et acétate.

9. Le procédé selon l'une quelconque des revendications 1 à 8, où l'étape d) est effectuée avec l'application de limitations cartésiennes sous le récepteur.

10. Le procédé selon l'une quelconque des revendications 1 à 9, dans lequel le récepteur macromoléculaire est une protéine ou une glycoprotéine.

11. Le procédé selon l'une quelconque des revendications 1 à 9, dans lequel le récepteur macromoléculaire est un acide nucléique.

12. Un produit de programme informatique comprenant des instructions de programme pour faire qu'un ordinateur effectue le procédé de chimie numérique pour calculer le(s) énergie(s) libre(s) de liaison d'une molécule se liant à un récepteur macromoléculaire tel que défini dans l'une quelconque des revendications 1 à 11.

13. Le produit de programme informatique selon la revendication 12, incorporé dans un support de stockage.

14. Le produit de programme informatique selon la revendication 12, porté dans un signal porteur.

15. Un procédé de chimie numérique pour calculer le(s) énergie(s) libre(s) de liaison d'une molécule se liant à un récepteur macromoléculaire en utilisant des données obtenues par des simulations classiques, qui sont effectuées dans un solvant explicite mélangé, comprenant :

a) un moyen informatique pour introduire la structure 3D d'un récepteur macromoléculaire dans un système informatique ;
b) un moyen informatique pour établir au moins un système de simulation co-solvaté comprenant le récepteur macromoléculaire et un mélange de solvants explicites comprenant de l'eau et au moins un co-solvant amphiphile organique;
c) un moyen informatique pour affecter des types d'atome aux atomes des molécules de solvant de l'au moins un système de simulation co-solvaté de l'étape b) ;
d) un moyen informatique pour calculer au moins une trajectoire de l'au moins un système de simulation co-solvaté ;
e) un moyen informatique pour partager l'au moins un système de simulation co-solvaté dans des éléments de volume ;
f) un moyen informatique pour compter la présence de chaque type d'atome de solvant dans chaque élément de volume le long de l'au moins une trajectoire de l'au moins un système de simulation co-solvaté pour donner le taux de remplissage total au cours de la période de simulation dans l'élément de volume ;
g) un moyen informatique pour utiliser les résultats de la dernière étape pour obtenir des énergies libres de liaison à la macromolécule des types d'atome différents trouvés dans les molécules de solvant ;
h) un moyen informatique pour découpler la contribution de chaque type d'atome de la molécule de co-solvant du reste de types d'atome de la molécule de co-solvant pour obtenir des énergies libres de liaison corrigées du type d'atome dans chaque élément de volume en effectuant une étape choisie dans le groupe constitué de :

h1) redistribuer, dans l'étape f), les unités de comptage parmi les types d'atome de la molécule de co-solvant de manière que le comptage affecté au type d'atome soit directement proportionnel à sa contribution à l'énergie libre de liaison ;
et

h2) soustraire, dans l'étape g), l'énergie libre de liaison due à tous les autres types d'atome présents dans la molécule de co-solvant de l'énergie libre de liaison non corrigée du type d'atome.

i) un moyen informatique pour recueillir toutes les énergies libres de liaison corrigées obtenues dans l'étape h) pour tous les types d'atome afin d'établir une fonction de score corrigée ; et

j) un moyen informatique pour calculer, sur la base de la méthode d'amarrage, l'énergie libre de liaison de la molécule se liant au récepteur macromoléculaire en utilisant la fonction de score corrigée obtenue dans l'étape i).

FIG.1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2010129386 A2

### Non-patent literature cited in the description

- **HANN MM.** Molecular complexity and its impact on the probability of finding leads for drug discovery. *J. Chem. Inf. Comput. Sci.,* 2001, vol. 41, 856-864
- **OROZCO M. ; LUQUE FJ.** Theoretical methods for the description of the solvent effect in biomolecular systems. *Chem. Rev,* 2000, vol. 100, 4187-4226
- **YOUNG T.** Dewetting transitions in protein cavities. *Proteins,* 2010, vol. 78, 1856-1869
- **YOUNG T.** Motifs for molecular recognition exploiting hydrophobic enclosure in protein-ligand binding. *PNAS,* 2006, vol. 104, 808-813
- **KUNTZ ID.** The maximal affinity of ligands. *PNAS,* 1999, vol. 96, 9997-10002
- **FERSHT AR.** The hydrogen bond in molecular recognition. *Trends in Biochemical Science,* vol. 12, 301-304
- **KARPLUS PA.** Hydrophobicity regained. *Protein Science,* 1997, vol. 6, 1302-1307
- **BEN-NAIM, A.** Molecular theory of solutions. Oxford University Press, September 2006
- **HUANG, N.** Benchmarking set for molecular docking. *J.Med.Chem,* 2006, vol. 16, 6789-6801
- **BAUM B.** Non-additivity of functional group contributions in protein-ligand binding: A comparative study by crystallography and isothermal titration calorimetry. *J. Mol. Biol.,* 2010, vol. 397, 1042-1054
- **BAUM B.** Non-additivity of functional group contributions in protein-ligand binding: A comparative study by crystallography and isothermal titration calorimetry. *J. Mol. Biol,* 2010, vol. 397, 1042-1054